# EUROPEAN PATENT APPLICATION

(11) **EP 4 124 656 A1**
(43) Date of publication of application: **01.02.2023**
(21) Application number: 21187801.2
(22) Date of filing: 26.07.2021
(51) Int. Cl.: C12N 15/113, A61K 31/713, A61P 35/00

(54) **PD-L1 MICRORNAS**

(71) Applicant: Martin-Luther-Universität Halle-Wittenberg, 06108 Halle (Saale) (DE)
(72) Inventor: Seliger, Barbara, 06114 Halle (DE); Vaxevanis, Christoforos, 06108 Halle (DE); Friedrich, Michael, 35037 Marburg (DE)
(74) Representative: Grünecker Patent- und Rechtsanwälte PartG mbB

(57) **Abstract**

The present invention relates to microRNAs and their use in therapy, particularly in the treatment of cancer. In particular, the invention relates to nucleic acids, micro RNAs and micro RNA mimics that are useful in treating PD-L1 positive cancer.

## Description

### TECHNICAL FIELD

The present disclosure generally relates to microRNAs and their use in therapy, particularly in the treatment of cancer.

### BACKGROUND OF THE INVENTION

Programmed death ligand 1 (PD-L1), also known as cluster of differentiation 274 (CD274) or B7-H1, is a type 1 transmembrane protein, which interacts with a T cell inhibitory receptor named programmed cell death protein-1 (PD1). The interaction between PD1 and PD-L1 on the cell surface of T cells promotes T cell tolerance, minimizes autoimmune mediated inflammation, but also results in an immune escape of tumor cells. PD-L1 is a checkpoint protein, which is expressed by various T cells and antigen presenting cells, but is also overexpressed in different cancer cells. Expression by cancer cells results in a reduced elimination of tumor cells by CD8+ cytotoxic T lymphocytes (CTL), thereby promoting tumor progression. Indeed, increased levels of PD-L1 expression have been associated with disease progression in cancer, high risk of cancer mortality as well as a poor patient outcome. Targeted therapies blocking the interaction between PD-L1 and PD-1 are a promising therapeutic option for various cancer types and already approved for the treatment of e.g. melanoma, non-small cell lung cancer (NSCLC), renal cell carcinoma (RCC), bladder cancer and head and neck squamous cell carcinoma (HNSCC).

Despite the promising results, the objective response rate of patients with various cancer types are still low. This might be due to the high individual variation of PD-L1 expression mediated by cellular and soluble components of the tumor microenvironment (TME) (Kim, J.M. and D.S. Chen, Ann Oncol, 2016. 27(8): p. 1492-504). Furthermore, it has been demonstrated that poor responders to immune checkpoint inhibitor (CPI) therapy express low PD-L1 levels and have a reduced T cell infiltration. Thus, there is a need to provide novel compositions targeting PD-L1.

Recently, a complex regulation of PD-L1 expression was reported including genomic alterations, transcriptional and epigenetic control, mRNA and protein stability, post-transcriptional regulation as well as inflammatory and oncogenic signaling.

### SUMMARY OF THE INVENTION

Here, novel PD-L1 regulating miRNAs interacting with the 3'-UTR (untranslated region) and/or CDS (coding sequences) of the PD-L1 gene are identified. These novel miRNAs suppress the expression and function of PD-L1 and can be used in a method of treatment of cancer patients, in particular melanoma, non-small cell lung cancer (NSCLC), renal cell carcinoma (RCC), bladder cancer and head and neck squamous cell carcinoma (HNSCC) cancer patients.

In a first aspect, the present invention provides a nucleic acid comprising a sequence that has at least 70%, at least 75%, at least 80%, at least 85%, at least 90%, at least 95% or 100% sequence identity with a sequence selected from the group consisting of the sequences according to any one of SEQ ID NOs: 1 to 60, preferably the sequence is selected from the group consisting of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6), more preferably the sequence is selected from the group consisting of hsa-miR-103b (SEQ ID NO: 4), hsa-miR-181b-5p (SEQ ID NO: 5) and miR-186-5p (SEQ ID NO: 6).

In an embodiment, the present invention provides nucleic acids comprising a sequence selected from the group consisting of SEQ ID NOs: 1-6 and SEQ ID NOs: 9-60 and uses thereof.

In an embodiment, the sequence comprised in the nucleic acid is selected from the group consisting of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6). Preferably, the sequence is selected from the group consisting of hsa-miR-103b (SEQ ID NO: 4), hsa-miR-181b-5p (SEQ ID NO: 5) and miR-186-5p (SEQ ID NO: 6).

In particular, the nucleic acid or a miRNA that is encoded by the nucleic acid is capable of hybridizing to a PD-L1 mRNA. Preferably, the nucleic acid or a miRNA that is encoded by the nucleic acid is capable of hybridizing to the 3' UTR and/or the CDS of a PD-L1 mRNA, more preferably wherein the miRNA binds both the 3' UTR and the CDS of a PD-L1 mRNA.

The nucleic acid or a miRNA that is encoded by the nucleic acid is capable of reducing gene expression of PD-L1, preferably wherein the nucleic acid or a miRNA that is encoded by the nucleic acid reduces translation of the PD-L1 mRNA.

In an embodiment, the nucleic acid is comprised in a vector. The nucleic acid may comprise a miRNA or the nucleic acid may be a miRNA. The nucleic acid may also be a miRNA mimic.

In another aspect, the invention provides a vector encoding the nucleic acid.

In a further aspect the invention provides a miRNA mimic comprising a sequence that has at least 70%, at least 75%, at least 80%, at least 85%, at least 90%, at least 95% or 100% sequence identity with a sequence selected from the group consisting of the sequences according to any one of SEQ ID NOs: 1 to 60.. Preferably the miRNA mimic is capable of hybridizing to a PD-L1 mRNA, and/or the miRNA mimic is capable of reducing gene expression of PD-L1, more preferably wherein the miRNA mimic reduces translation of the PD-L1 mRNA.

In a further aspect, the invention provides a cell comprising the nucleic acid, the vector or the miRNA mimic. The cell may be an immune cell, preferably a CAR T cell.

In a further aspect, the invention provides the nucleic acid, the vector, the miRNA mimic or the cell for use in therapy. In a preferred embodiment, the nucleic acid, the vector, the miRNA mimic or the cell is for use in treating cancer. Preferred is the treatment of skin cancer (e.g. melanoma), non-small cell lung cancer (NSCLC), Hodgkin's lymphoma, bladder cancer, renal cell carcinoma (RCC), head and neck squamous cell carcinoma (HNSCC), breast cancer, Merkel cell carcinoma, hepatocellular carcinoma (HCC) or gastric cancer (GC). More preferably the cancer is skin cancer, e.g. melanoma, non-small cell lung cancer (NSCLC), renal cell carcinoma (RCC), bladder cancer or head and neck squamous cell carcinoma (HNSCC). Most preferably, the cancer is skin cancer, in particular melanoma. In particular, the cancer is a cancer that is characterized by the overexpression of PD1 on the cell surface of tumor cells.

In a further aspect, the invention provides the use of the nucleic acid, the vector, the miRNA mimic or the cell for silencing PD-L1 gene expression. Preferably, the miRNA is hsa-miR-155-5p (SEQ ID NO: 7), hsa-miR-17-5p (SEQ ID NO: 8) or a combined application of the two.

In a further aspect, a pharmaceutical composition comprising the nucleic acid, the vector, the miRNA mimic or the cell in combination with a pharmaceutically acceptable carrier or excipient is provided.

In a further aspect, a method is provided of treating a subject having a cancer, the method comprising the step of administering to the subject an effective amount of the nucleic acid, the vector or the miRNA mimic, thereby treating the cancer. The administration of the nucleic acid, the vector or the miRNA mimic reduces gene expression of PD-L1, preferably in the cancer cells of the cancer to be treated. In one embodiment, the nucleic acid or miRNA mimic comprises at least one of the following sequences hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) or hsa-miR-186-5p (SEQ ID NO: 6).

In an embodiment the sequence that is comprised in the nucleic acid, the miRNA, the miRNA mimic, the vector, or the cell, selected from the group consisting of the sequences according to any one of SEQ ID NOs: 1-60, differs from the anyone sequence of SEQ ID NOs: 1-60 in 0, 1, 2, 3, or 4 nucleotides. If the sequence differs in 0 nucleotides, the comprised sequence is identical to the anyone sequence of SEQ ID NOs: 1-60. If the sequence differs in 1, 2, 3, or 4 nucleotides, this means that the sequence that is comprised in the nucleic acid, the miRNA, the miRNA mimic, the vector, or the cell may differ from the anyone sequence of SEQ ID NOs: 1-60 by 1, 2, 3, or 4 nucleotides. A sequence differs by 1, 2, 3, or 4 nucleotides if the number of nucleotides of the comprised sequence is identical to the number of nucleotides of the anyone sequence of SEQ ID NOs: 1-60, however, the identity of 1, 2, 3, or 4 nucleotides of the nucleotides of the comprised sequence is changed relative to the identity of the nucleotides of the anyone sequence of SEQ ID NOs: 1-60. The identity of the nucleotides may be changed from A to G, from A to C, or from A to U, from G to A, from G to C, or from G to U, from C to A, from C to G, or from C to U, from U to A, from U to G, or from U to C, wherein A stands for adenylate, G stands for guanylate, C stands for cytidylate and U stands for uracilylate. The above-mentioned changes includes changes from A, G, C or U to the corresponding mimic as indicated elsewhere herein. A sequence also differs by 1, 2, 3, or 4 nucleotides if 1, 2, 3, or 4 nucleotides are missing relative to the anyone sequence of SEQ ID NOs: 1-60, at the 5'-end, the 3'-end or between the 5'-end and the 3'-end. In some embodiments differing may also mean that certain nucleotides are missing other nucleotides are changed as indicated above, as long as the absolute difference relative to the anyone sequence of SEQ ID NOs: 1-60 the not exceed 4 nucleotides. The present uses and methods are generally contemplated for in vitro and in vivo use. For instance, the nucleic acid, the miRNA or the miRNA mimic may be used for gene silencing in vitro and in vivo.

### BRIEF DESCRIPTION OF DRAWINGS

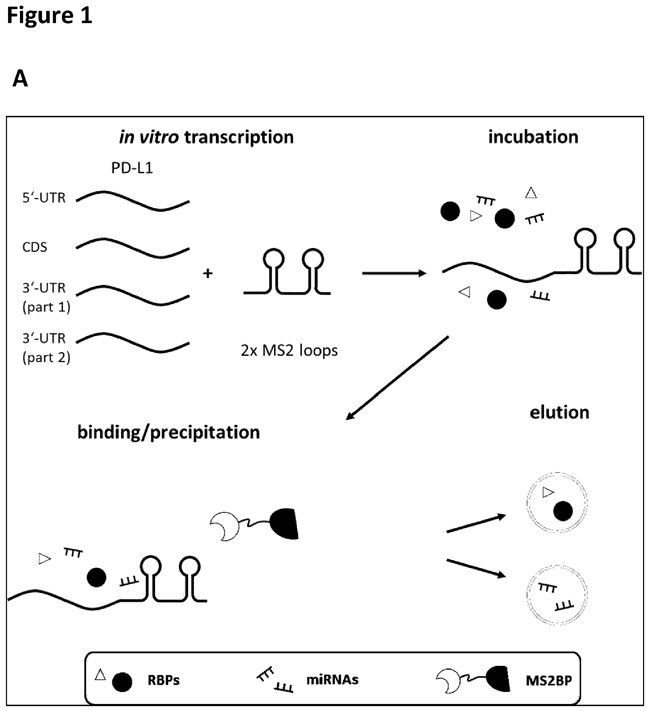

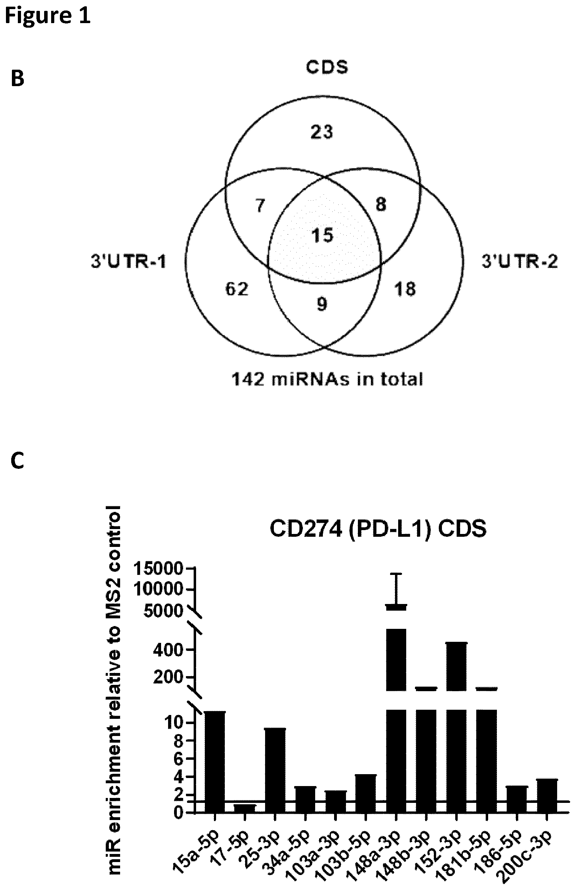

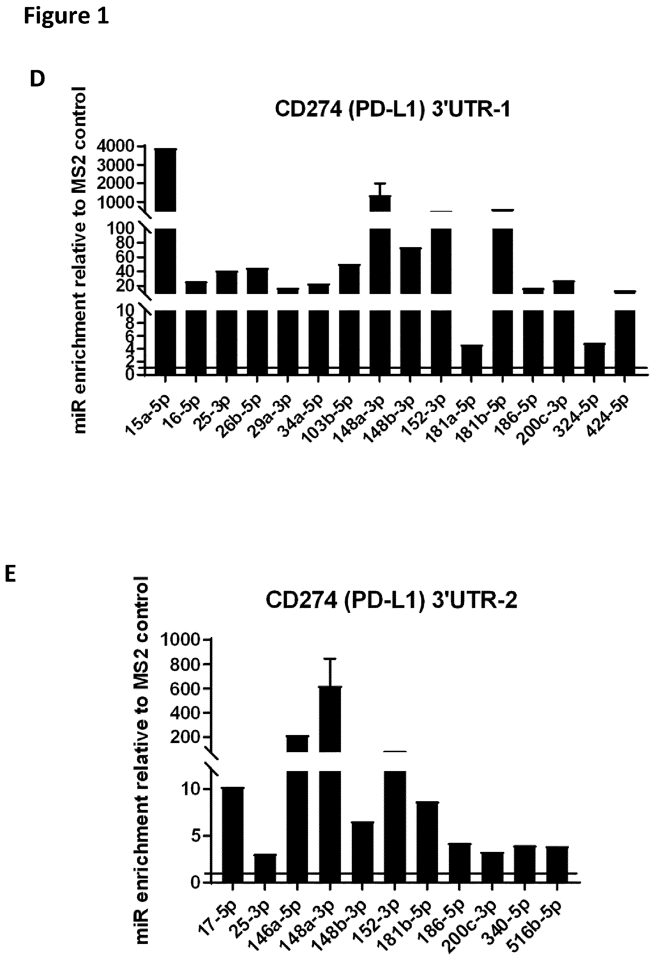

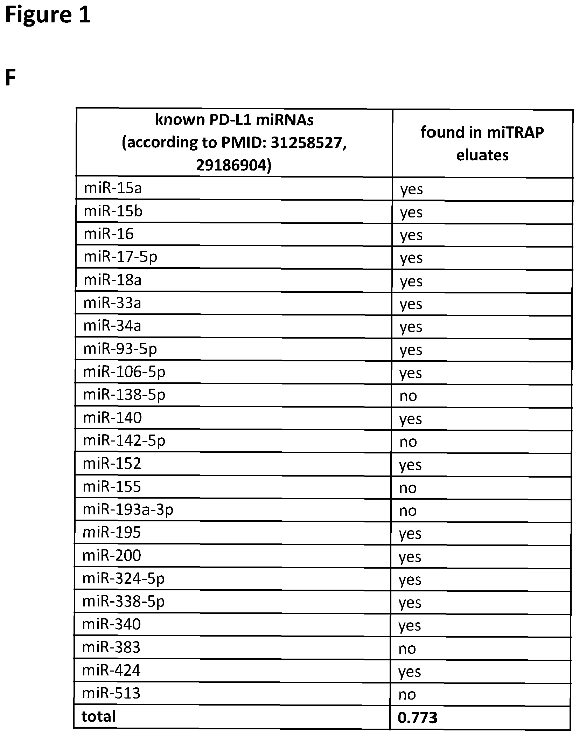

**Figure 1****.** Identification of PD-L1 targeting miRs. **A)** Graphical representation of the miTRAP method followed for the identification of PD-L1 specific miRNAs. **B)** Venn diagram of miRNAs identified relative to their binding to the three different regions of PD-L1 mRNA, represented as enrichment compared to the MS2 control. **C-E)** Validation of selected candidates by qPCR. The relative expression of the target miRNAs is depicted in comparison to the enrichment found in the MS2 control, which is set to 1 (black line). **F)** Known miRNAs found in miTRAP eluates.

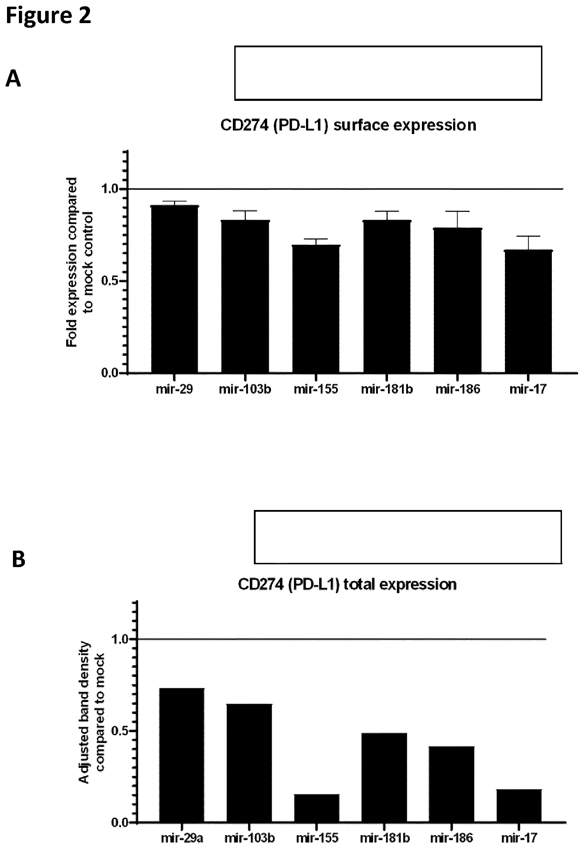

**Figure 2****.** PD-L1 expression and miRNA mimics. The relative surface expression (A) and total expression **(B)** of PD-L1 after transfection with miRNA mimics when compared to the mock control (horizontal line), as determined by flow cytometry and Western blot analysis.

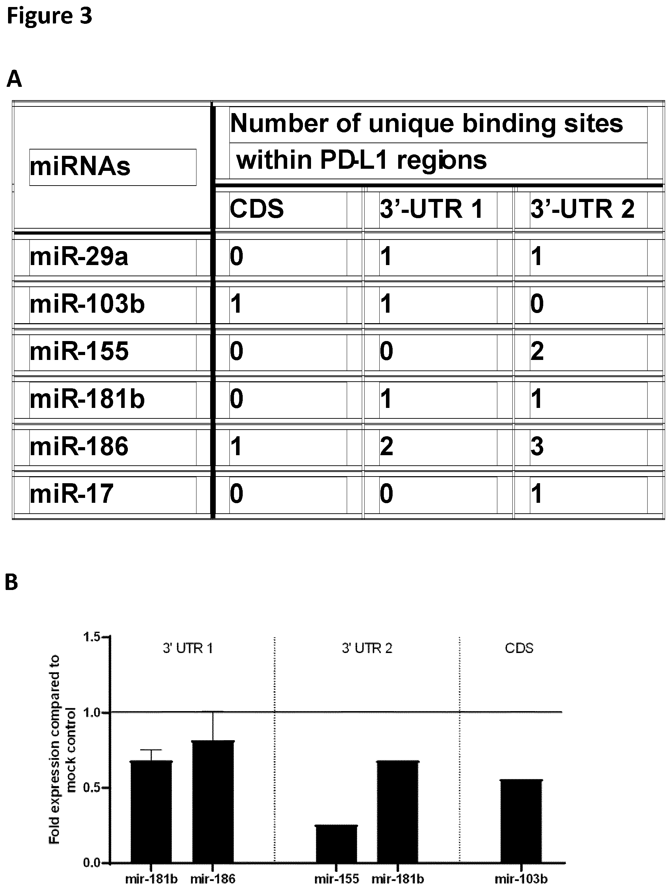

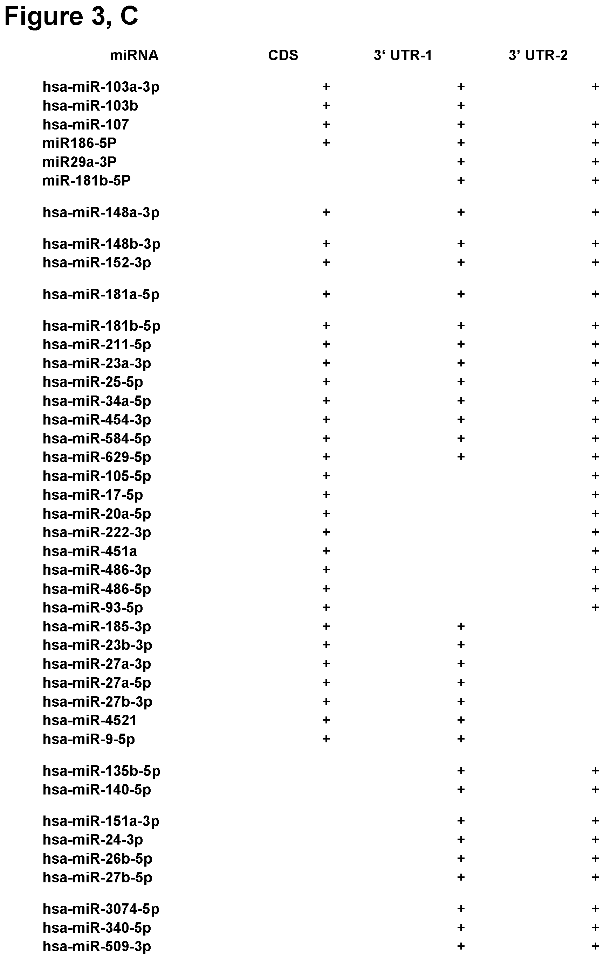

**Figure 3**. A)Number of putative binding sites of miRNAs in 3 different regions of PD-L1 mRNA. Binding sites were predicted through Target Scan 7.2, a miRNA prediction tool. **B)** Relative luminescence levels after transfection with miRNA mimics compared to the mock control (horizontal line), as assessed by dual luciferase assay. **C)** Location of the binding site of the miRNAs in the PD-L1 regions CDS, 3'UTR-1 and 3'UTR-2.

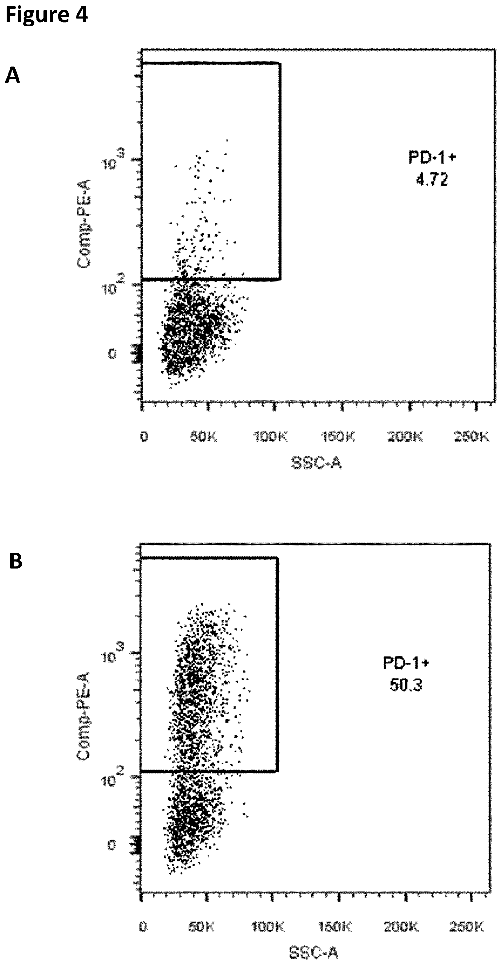

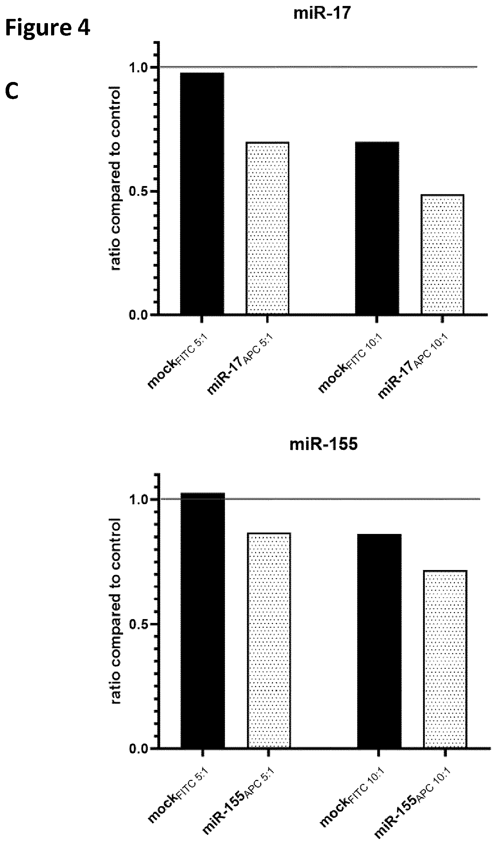

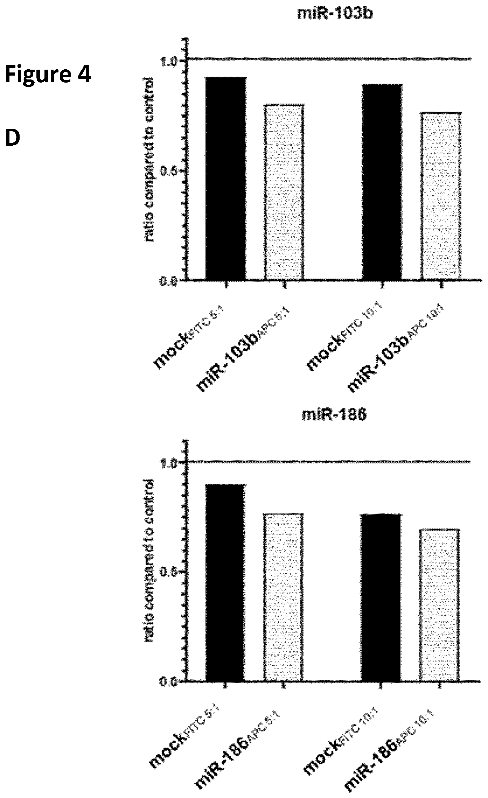

**Figure 4****.** Effect of PD-L1 miRNAs on T cell responses. PD1 expression on CD8⁺ T cells left untreated **(A)** or stimulated for 24 h with the cytokine cocktail after the activation protocol **(B).** Shown are two representative plots from PBMC stimulated with the DC/IL2 out of 3 different experiments. **C-D)** Mixture of FITC-labeled mock control (filled bars) and APC-labeled miRNA-transfected (dotted bars) mel1359 cells were co-cultured with antigen specific stimulated CD8⁺ T cells. After 4 h co-culture the amount of live tumor cells was evaluated upon live aqua staining. Shown are the ratio of live mock, miR-17, miR-155, miR-103b and miR-186 transfected at the 5:1 and 10:1 E:T ratio versus the control (no effector cells).

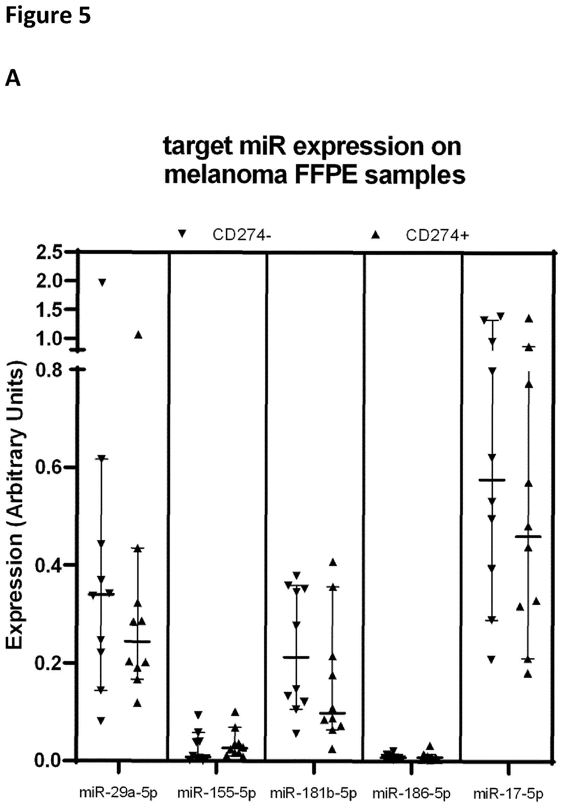

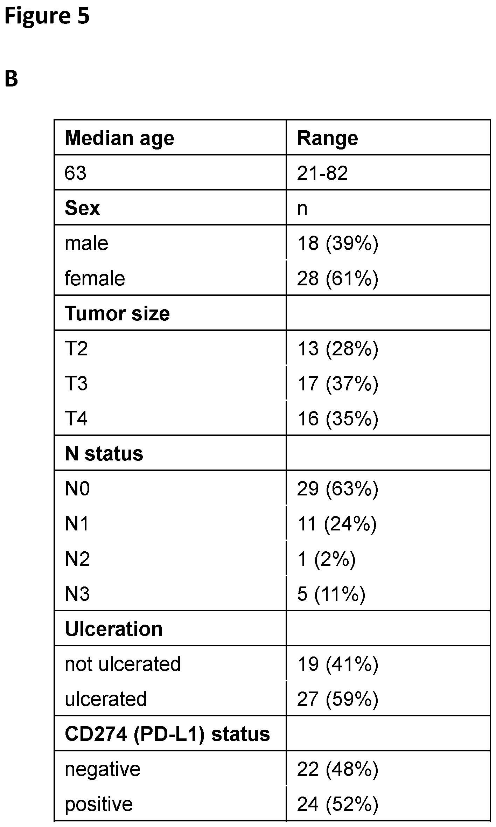

**Figure 5****.** Determination of the clinical relevance of miRNAs. **A)** Comparison of the expression of miRNA candidates in FFPE samples of melanoma patients with low or high PD-L1 expression. **B)** Clinicopathological characteristics of the melanoma patients.

### DETAILED DESCRIPTION OF THE INVENTION

### Definitions

Unless defined otherwise, all technical and scientific terms used herein have the meaning commonly understood by the person skilled in the art.

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features, in addition to the explicitly stated technical features. In the same sense, the term "involving" as well as other respective grammatical forms such as "involves" and "involved" is not limiting. The same applies for the term "including" and other grammatical forms such as "includes" and "included".

Section headings throughout the description are for organizational purposes only. In particular, they are not intended as limiting for various embodiments described therein, and it is to be understood that embodiments (and features therein) described under one subheading may be freely combined with embodiments (and features therein) described under another subheading.

Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

As used herein, the term "nucleic acid" refers to an oligomer or polymer of naturally occurring or modified nucleotides. Nucleotides include ribonucleotides and deoxyribonucleotides. The nucleic acid includes oligomers and polymers comprising naturally occurring nucleotides, oligomers or polymers comprising naturally occurring nucleotides and modified nucleotides, and oligomers or polymers comprising modified nucleotides. The nucleic acid may also comprise nucleotide substitutions relative to a reference sequence. For instance, the nucleic acid may contain 1, 2, 3, 4, 5, 6, 7, 8, 9 or 10 nucleotide substitutions. The nucleic acid may have 100 %, 95%, 90%, 85%, or 80% sequence identity to a reference sequence. A reference sequence may be a nucleic acid with a sequence according to SEQ ID NO: 1 to SEQ ID NO: 60. Modified or substituted nucleotides may be preferred over naturally occurring nucleotides, because of properties of the modified or substituted nucleotides such as enhanced stability in the presence of nucleases. A nucleic acid may be a ribonucleic acid (RNA) or a deoxyribonucleic acid (DNA) or a hybrid between RNA and DNA. The Nucleic acid may be single or double stranded. The double stranded nucleic acid may be RNA-RNA, RNA-DNA (hybrid) or DNA-DNA. Double strand formation occurs through for example, hydrogen bonds according to the Watson-Crick-base pairing. The nucleic acid may have one or two 3' overhangs. The nucleic acid may have one or two 5' overhangs. The nucleic acid may have one or two blunt ends. Combinations of the above-referenced overhangs or blunt ends are also included.

The nucleic acid can be defined by a sequence of nucleotides indicated in the commonly accepted letter code for the base of the nucleotide, A (adenine), C (cytosine), G (guanine), T (thymine) and U (uracil). "Sequence" as used herein refers to a sequence of nucleotides. An indicated sequence includes the indicated sequence of naturally occurring nucleotides and modifications thereof, for instance a miRNA mimic.

As used herein, the term "microRNA (miRNA)" refers to a small single stranded RNA nucleic acid with a length of 17-27 nucleotides, preferably 22 nucleotides, which is capable of causing gene silencing of a target gene when the miRNA is present within a cell and which comprises a sequence that is complementary to a portion of the messenger RNA (mRNA) of the target gene. The miRNA may have a length of 17, 18, 19, 20, 21, 22, 23, 24, 25, 26, or 27 nucleotides. Preferred are miRNAs with a length of 20, 21, 22, 23 or 24 nucleotides. Particularly preferred are miRNAs with a length of 22 nucleotides. The miRNA may be a pri-miRNA, a pre-miRNA a mature miRNA or any other intermediate structure commonly known to the skilled person.

As used herein, the term "miRNA mimic" refers to a modified, small, partially or completely double-stranded nucleic acid, which has the same function as a corresponding miRNA, i.e. which is capable of causing gene silencing of the target gene of the corresponding miRNA when the miRNA mimic is present within a cell and which comprises a sequence that is complementary to a portion of the messenger RNA (mRNA) of the target gene. In a miRNA mimic, the sugar moiety, phosphate and/or the base of one or more nucleotides is chemically modified, in particular covalently modified. The two strands of the miRNA mimic are referred to as "first strand" and "second strand", wherein the first strand is complementary to a portion of the mRNA of the target gene. The miRNA mimic has a length a length of about 22 nucleotides, preferably a length of 17-27 nucleotides. The miRNA mimic may have a length of 17, 18, 19, 20, 21, 22, 23, 24, 25, 26, or 27 nucleotides. Preferred are miRNA mimics with a length of 20, 21, 22, 23 or 24 nucleotides. Particularly preferred are miRNA mimics with a length of 22 nucleotides. A miRNA mimic may be defined by the sequence of nucleotides and modified nucleotides comprised in the miRNA mimic. The miRNA mimic may also consist of a sequence of first strand nucleotides and modified nucleotides as disclosed herein in combination with a corresponding second strand.

Throughout the disclosure, the term "microRNA mimic" may be used interchangeably with the terms "promiR," "miR agonist," "microRNA agonist," "microRNA mimetic," "miRNA mimic," or "miR mimic".

The term "first strand" may be used interchangeably with the terms "antisense strand" or "guide strand". The term "second strand" may be used interchangeably with the term "sense strand" or "passenger strand".

The term "seed", also referred to as "seed sequence" or "seed region", is a 6-mer sequence located within positions 2-8 as counted from the miRNA 5'-end of the miRNA. Even though base pairing between the entire miRNA and its target mRNA does not have to be perfect for miRNA mediated gene silencing to occur, the "seed sequence" has to be completely complementary to the corresponding mRNA sequence.

The term "vector" as used herein is a molecular construct that comprises a nucleic acid, generated recombinantly or synthetically, comprising one or more functional genetic elements, which is capable of delivering a nucleic acid into a cell and is capable of expressing a nucleic acid and/or integrating a nucleic acid into the genome of the cell. In particular, a vector, upon entering a target cell, may express the miRNA. Typically, the nucleic acid sequence to be expressed is under the control of genetic elements, such as a constitutive or inducible promoter. Vectors can be used to transiently or stably express the miRNA in a cell.

As used herein, the term "capable of hybridizing" means capable of hybridizing under stringent conditions, for instance, under conditions that correspond to a Tm (melting temperature) equal to or greater than about 50°C, 51°C, 53°C,54°C, 55°C, 56°C, 57°C, 58°C, 59°C, 60°C, 61°C, 62°C, 63°C, 64°C, 65°C, 66°C, 67°C, 68°C, 69°C or 70°C.

A nucleic acid comprising a sequence or a nucleic acid consisting of a sequence

"Gene silencing" as used herein refers to the regulation of gene expression that leads to a reduction of the number of gene transcripts (mRNA) and/or a reduction of the number of translated gene products in a cell.

As used herein, the term "modified" and the corresponding noun "modification" refers to chemical modifications, in particular covalent modifications, of the sugar moiety, phosphate and/or the base of a nucleotide of the nucleic acid, miRNA or miRNA mimic. In particular, the 2'-hydroxyl of the ribose can be modified to protect the nucleic acid from degradation. Different chemical modifications have been developed such as phosphodiester linkages, ribose backbone, 2'-O-(2-Methoxyethyl), 2'-O-Methyl, 2'- locked nucleic acid, and 2'- Fluoro.

As used herein, the term "subject" refers to a mammal, without limitation, including humans and other primates (e.g., chimpanzees, cynomologous monkeys, and other apes and monkey species), farm animals (e.g., cattle, sheep, pigs, goats and horses), domestic mammals (e.g., dogs and cats), laboratory animals (e.g., rabbits, rodents such as mice, rats, and guinea pigs), and birds (e.g., domestic, wild and game birds such as chickens, turkeys and other gallinaceous birds, ducks, geese, and the like). In preferred embodiments, the subject is a human.

### List of abbreviations

BSA, bovine serum albumin; CDS, coding sequence; CD274, cluster of differentiation 274; CPI, checkpoint inhibitor; CTL, cytotoxic T lymphocyte; CTLA-4, cytotoxic T lymphocyte-associated protein 4; DFS, disease-free survival; E:T, effector to target tumor cell; FFPE, formalin-fixed paraffin-embedded; HLA, human leukocyte antigen; HNSCC, head and neck squamous cell carcinoma; HRP, horseradish peroxidase; luc, luciferase; mAb, monoclonal antibody; MFI, mean fluorescence intensity; microRNA, miRNA; miTRAP, miRNA trapping by RNA *in vitro* affinity purification; NSCLC, non-small cell lung cancer; OS, overall survival; PBMC, peripheral blood mononuclear cell; PDCD1/PD1, programmed cell death-1; PD-L1, programmed death ligand 1; PDM, PD-L1 microRNA; RCC, renal cell carcinoma; TGF-β, transforming growth factor beta; TGFBR2, transforming growth factor beta receptor 2; TME, tumor microenvironment; TPM, normalized read counts; 3'-UTR, 3' untranslated region.

All publications, including but not limited to patents, patent applications and scientific publications, cited in this description are herein incorporated by reference for all purposes as if each individual publication were specifically and individually indicated to be incorporated by reference.

### Nucleic acids

In a first aspect, the invention provides a nucleic acid comprising a sequence that has at least 70%, at least 75%, at least 80%, at least 85%, at least 90%, at least 95% or 100% sequence identity with a sequence selected from the group consisting of the sequences according to any one of SEQ ID NOs: 1 to 60, preferably the sequence is selected from the group consisting of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6), more preferably the sequence is selected from the group consisting of hsa-miR-103b (SEQ ID NO: 4), hsa-miR-181b-5p (SEQ ID NO: 5) and miR-186-5p (SEQ ID NO: 6).

In a preferred embodiment, the nucleic acid comprises a sequence selected from the group consisting of the sequences according to any one of SEQ ID NOs: 1 to 60, preferably the sequence is selected from the group consisting of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6), more preferably the sequence is selected from the group consisting of hsa-miR-103b (SEQ ID NO: 4), hsa-miR-181b-5p (SEQ ID NO: 5) and miR-186-5p (SEQ ID NO: 6).

In an embodiment, the nucleic acid or a miRNA that is encoded by the nucleic acid is capable of hybridizing to a PD-L1 mRNA.

The nucleic acid may be a synthetic nucleic acid, preferably, the nucleic acid is a synthetic RNA molecule. More preferably, the nucleic acid is a synthetic double stranded RNA molecule.

In an embodiment the nucleic acid comprises one or more of the sequences according to Table 1.

In a preferred embodiment, the nucleic acid consists of any one of the sequences according to Table 1. In an embodiment, the nucleic acid comprises a sequence selected from the group consisting of the sequences according to Table 1, except hsa-miR-155-5p and/or hsa-miR-17-5p.

In a preferred embodiment the nucleic acid of the invention is an isolated nucleic acid. An isolated nucleic acid is a nucleic acid outside a cell and/or organism, such as a human. An isolated nucleic acid may be chemically synthesized or produced by recombinant production in cells and subsequently harvested from these cells, such as bacterial or mammalian cells.

In a preferred embodiment, the nucleic acid of the invention comprises the sequence of hsa-miR-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) or hsa-miR-186-5p (SEQ ID NO: 6) and/or any combination thereof.

In a more preferred embodiment, the nucleic acid of the invention comprises the sequence of hsa-miR-29a-3p (SEQ ID NO: 3), hsa-miR-103b (SEQ ID NO: 4), hsa-miR-181b-5p (SEQ ID NO: 5) or hsa-miR-186-5p (SEQ ID NO: 6) and/or any combination thereof.

**Table 1: miRNA sequences**

| **miRNA name** | **Sequence of first strand** | **SEQ ID NOs** |
|---|---|---|
| hsa-mir-26a-5p | UUCAAGUAAUCCAGGAUAGGCU | SEQ ID NO: 1 |
| hsa-miR-26b-5p | UUCAAGUAAUUCAGGAUAGGU | SEQ ID NO: 2 |
| hsa-miR-29a-3p | UAGCACCAUCUGAAAUCGGUUA | SEQ ID NO: 3 |
| hsa-miR-103b | UCAUAGCCCUGUACAAUGCUGCU | SEQ ID NO: 4 |
| hsa-miR-181b-5p | AACAUUCAUUGCUGUCGGUGGGU | SEQ ID NO: 5 |
| hsa-miR-186-5p | CAAAGAAUUCUCCUUUUGGGCU | SEQ ID NO: 6 |
| hsa-miR-155-5p | UUAAUGCUAAUCGUGAUAGGGGUU | SEQ ID NO: 7 |
| hsa-miR-17-5p | CAAAGUGCUUACAGUGCAGGUAG | SEQ ID NO: 8 |
| hsa-miR-140-5p | CAGUGGUUUUACCCUAUGGUAG | SEQ ID NO: 9 |
| hsa-miR-425-5p | AAUGACACGAUCACUCCCGUUGA | SEQ ID NO: 10 |
| hsa-miR-30b-5p | UGUAAACAUCCUACACUCAGCU | SEQ ID NO: 11 |
| hsa-miR-574-3p | CACGCUCAUGCACACACCCACA | SEQ ID NO: 12 |
| hsa-miR-29b-3p | UAGCACCAUUUGAAAUCAGUGUU | SEQ ID NO: 13 |
| hsa-miR-9-5p | UCUUUGGUUAUCUAGCUGUAUGA | SEQ ID NO: 14 |
| hsa-miR-320b | AAAAGCUGGGUUGAGAGGGCAA | SEQ ID NO: 15 |
| hsa-miR-25-3p | CAUUGCACUUGUCUCGGUCUGA | SEQ ID NO: 16 |
| hsa-miR-103a-3p | AGCAGCAUUGUACAGGGCUAUGA | SEQ ID NO: 17 |
| hsa-miR-320c | AAAAGCUGGGUUGAGAGGGU | SEQ ID NO: 18 |
| hsa-miR-629-5p | UGGGUUUACGUUGGGAGAACU | SEQ ID NO: 19 |
| hsa-miR-24-3p | UGGCUCAGUUCAGCAGGAACAG | SEQ ID NO: 20 |
| hsa-miR-3074-5p | GUUCCUGCUGAACUGAGCCAG | SEQ ID NO: 21 |
| hsa-miR-532-3p | CCUCCCACACCCAAGGCUUGCA | SEQ ID NO: 22 |
| hsa-miR-181a-5p | AACAUUCAACGCUGUCGGUGAGU | SEQ ID NO: 23 |
| hsa-miR-27b-3p | UUCACAGUGGCUAAGUUCUGC | SEQ ID NO: 24 |
| hsa-miR-30c-5p | UGUAAACAUCCUACACUCUCAGC | SEQ ID NO: 25 |
| hsa-miR-151a-3p | CUAGACUGAAGCUCCUUGAGG | SEQ ID NO: 26 |
| hsa-miR-4521 | GCUAAGGAAGUCCUGUGCUCAG | SEQ ID NO: 27 |
| hsa-miR-106b-3p | CCGCACUGUGGGUACUUGCUGC | SEQ ID NO: 28 |
| hsa-miR-23a-3p | AUCACAUUGCCAGGGAUUUCC | SEQ ID NO: 29 |
| hsa-miR-20a-5p | UAAAGUGCUUAUAGUGCAGGUAG | SEQ ID NO: 30 |
| hsa-miR-146b-5p | UGAGAACUGAAUUCCAUAGGCUG | SEQ ID NO: 31 |
| hsa-miR-3074-5p | GUUCCUGCUGAACUGAGCCAG | SEQ ID NO: 32 |
| hsa-miR-30e-5p | UGUAAACAUCCUUGACUGGAAG | SEQ ID NO: 33 |
| hsa-miR-222-3p | AGCUACAUCUGGCUACUGGGU | SEQ ID NO: 34 |
| hsa-miR-340-5p | UUAUAAAGCAAUGAGACUGAUU | SEQ ID NO: 35 |
| hsa-miR-584-5p | UUAUGGUUUGCCUGGGACUGAG | SEQ ID NO: 36 |
| hsa-miR-550a-3-5p | AGUGCCUGAGGGAGUAAGAG | SEQ ID NO: 37 |
| hsa-miR-550a-5p | AGUGCCUGAGGGAGUAAGAGCCC | SEQ ID NO: 38 |
| hsa-miR-454-3p | UAGUGCAAUAUUGCUUAUAGGGU | SEQ ID NO: 39 |
| hsa-let-7g-5p | UGAGGUAGUAGUUUGUACAGUU | SEQ ID NO: 40 |
| hsa-miR-122-5p | UGGAGUGUGACAAUGGUGUUUG | SEQ ID NO: 41 |
| hsa-miR-3591-3p | AAACACCAUUGUCACACUCCAC | SEQ ID NO: 42 |
| hsa-miR-27a-3p | UUCACAGUGGCUAAGUUCCGC | SEQ ID NO: 43 |
| hsa-miR-185-3p | AGGGGCUGGCUUUCCUCUGGUC | SEQ ID NO: 44 |
| hsa-miR-25-5p | AGGCGGAGACUUGGGCAAUUG | SEQ ID NO: 45 |
| hsa-let-7e-5p | UGAGGUAGGAGGUUGUAUAGUU | SEQ ID NO: 46 |
| hsa-miR-211-5p | UUCCCUUUGUCAUCCUUCGCCU | SEQ ID NO: 47 |
| hsa-miR-221-3p | AGCUACAUUGUCUGCUGGGUUUC | SEQ ID NO: 48 |
| hsa-let-7f-5p | UGAGGUAGUAGAUUGUAUAGUU | SEQ ID NO: 49 |
| hsa-miR-128-3p | UCACAGUGAACCGGUCUCUUU | SEQ ID NO: 50 |
| hsa-miR-93-5p | CAAAGUGCUGUUCGUGCAGGUAG | SEQ ID NO: 51 |
| hsa-let-7i-5p | UGAGGUAGUAGUUUGUGCUGUU | SEQ ID NO: 52 |
| hsa-let-7c-5p | UGAGGUAGUAGGUUGUAUGGUU | SEQ ID NO: 53 |
| hsa-miR-378a-3p | ACUGGACUUGGAGUCAGAAGGC | SEQ ID NO: 54 |
| hsa-miR-105-5p | UCAAAUGCUCAGACUCCUGUGGU | SEQ ID NO: 55 |
| hsa-let-7b-5p | UGAGGUAGUAGGUUGUGUGGUU | SEQ ID NO: 56 |
| hsa-miR-193a-5p | UGGGUCUUUGCGGGCGAGAUGA | SEQ ID NO: 57 |
| hsa-miR-451a | AAACCGUUACCAUUACUGAGUU | SEQ ID NO: 58 |
| hsa-miR-103a-2-5p | AGCUUCUUUACAGUGCUGCCUUG | SEQ ID NO: 59 |
| hsa-miR-550b-3p | UCUUACUCCCUCAGGCACUG | SEQ ID NO: 60 |

In another embodiment, the nucleic acid comprises a sequence selected from the group consisting of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6) and/or any combination thereof.

In a preferred embodiment, the nucleic acid comprises a sequence selected from the group consisting of hsa-miR-103b (SEQ ID NO: 4), hsa-miR-181b-5p (SEQ ID NO: 5) and hsa-miR-186-5p (SEQ ID NO: 6).

In an embodiment, the nucleic acid is a double stranded nucleic acid. In a more preferred embodiment the double stranded nucleic acid is a double stranded RNA molecule. In an embodiment the nucleic acid is a RNA molecule in the form of a primary miRNA (pri-miRNA) that can be processed by the class 2 ribonuclease III enzyme Drosha. In a further embodiment, the nucleic acid is in the form of a pre-miRNA containing a 60-120 nt RNA hairpin in which one of the two strands includes the mature miRNA, preferably a miRNA of Table 1. In a further embodiment, the nucleic acid comprises a miRNA precursor. In a preferred embodiment, the precursor is approximately 70 nucleotides long.

In a preferred embodiment, the nucleic acid is a miRNA, more preferably a miRNA selected from the group of miRNAs of Table 1.

In an embodiment, the nucleic acid is capable of hybridizing to a PD-L1 mRNA. In an embodiment, the nucleic acid is capable of hybridizing to one or more regions of the PD-L1 transcript as shown in any of SEQ ID NOs: 61 to 64 in Table 2.

**Table 2. Full PD-L1 RNA sequence (transcript variant 1)**

| >NM_014143.4 Homo sapiens CD274 molecule (CD274), transcript variant 1, mRNA | | |
|---|---|---|
| **Region** | **Sequence** | **SEQ ID NO** |
| 5'UTR | agttctgcgcagcttcccgaggctccgcaccagccgc gcttctgtccgcctgcagggcattccagaaag | SEQ ID NO: 61 |
| CDS | atgaggatatttgctgtctttatattcatgacctact ggcatttgctgaacgcatttactgtcacggttcccaa ggacctatatgtggtagagtatggtagcaatatgaca | SEQ ID NO: 62 |
| | attgaatgcaaattcccagtagaaaaacaattagacc tggctgcactaattgtctattgggaaatggaggataa gaacattattcaatttgtgcatggagaggaagacctg aaggttcagcatagtagctacagacagagggcccggc tgttgaaggaccagctctccctgggaaatgctgcact tcagatcacagatgtgaaattgcaggatgcaggggtg taccgctgcatgatcagctatggtggtgccgactaca agcgaattactgtgaaagtcaatgccccatacaacaa aatcaaccaaagaattttggttgtggatccagtcacc tctgaacatgaactgacatgtcaggctgagggctacc ccaaggccgaagtcatctggacaagcagtgaccatca agtcctgagtggtaagaccaccaccaccaattccaag agagaggagaagcttttcaatgtgaccagcacactga gaatcaacacaacaactaatgagattttctactgcac ttttaggagattagatcctgaggaaaaccatacagct gaattggtcatcccagaactacctctggcacatcctc caaatgaaaggactcacttggtaattctgggagccat cttattatgccttggtgtagcactgacattcatcttc cgtttaagaaaagggagaatgatggatgtgaaaaaat gtggcatccaagatacaaactcaaagaagcaaagtga tacacatttggaggagacgtaa | |
| 3'UTR-1 | tccagcattggaacttctgatcttcaagcagggattctca acctgtggtttaggggttcatcggggctgagcgtgacaag aggaaggaatgggcccgtgggatgcaggcaatgtgggact taaaaggcccaagcactgaaaatggaacctggcgaaagca gaggaggagaatgaagaaagatggagtcaaacagggagcc tggagggagaccttgatactttcaaatgcctgaggggctc atcgacgcctgtgacagggagaaaggatacttctgaacaa ggagcctccaagcaaatcatccattgctcatcctaggaag acgggttgagaatccctaatttgagggtcagttcctgcag aagtgccctttgcctccactcaatcctcaatttgttttct gcatgactgagagtctcagtgttggaacgggacagtattt atgtatgagtttttcctatttattttgagtctgtgaggtc ttcttgtcatgtgagtgtggttgtgaatgatttcttttga agatatattgtagtagatgttacaattttgtcgccaaact aaacttgctgcttaatgatttgctcacatctagtaaaaca tggagtatttgtaaggtgcttggtctcctctataactaca agtatacattggaagcataaagatcaaaccgttggttgca taggatgtcacctttatttaacccattaatactctggttg acctaatcttattctcagacctcaagtgtctgtgcagtat ctgttccatttaaatatcagctttacaattatgtggtagc ctacacacataatctcatttcatcgctgtaaccaccctgt tgtgataaccactattattttacccatcgtacagctgagg aagcaaacagattaagtaacttgcccaaaccagtaaatag cagacctcagactgccacccactgtccttttataatacaa tttacagctatattttactttaagcaattcttttattcaa aaaccatttattaagtgcccttgcaatatcaatcgctgtg ccaggcattgaatctacagatgtgagcaagacaaagtacc tgtcctcaaggagctcatagtataatgaggagattaacaa gaaaatgtattattacaatttagtccagtgtcatagcata aggatgatgcgaggggaaaacccgagcagtgttgccaaga ggaggaaataggccaatgtggtctgggacggttggatata cttaaacatcttaataatcagagtaattttcatttacaaa gagaggtcggtacttaaaataaccctgaaaaataacactg gaattccttttctagcattatatttattcctgatttgcct | SEQ ID NO: 63 |
| | ttgccatataatctaatgcttgtttatatagtgtctggta ttgtttaacagttctgtct | |
| 3' UTR-2 | ggtacttaaaataaccctgaaaaataacactggaattcct tttctagcattatatttattcctgatttgcctttgccata taatctaatgcttgtttatatagtgtctggtattgtttaa cagttctgtcttttctatttaaatgccactaaattttaaa ttcatacctttccatgattcaaaattcaaaagatcccatg ggagatggttggaaaatctccacttcatcctccaagccat tcaagtttcctttccagaagcaactgctactgcctttcat tcatatgttcttctaaagatagtctacatttggaaatgta tgttaaaagcacgtatttttaaaatttttttcctaaatag taacacattgtatgtctgctgtgtactttgctatttttat ttattttagtgtttcttatatagcagatggaatgaatttg aagttcccagggctgaggatccatgccttctttgtttcta agttatctttcccatagcttttcattatctttcatatgat ccagtatatgttaaatatgtcctacatatacatttagaca accaccatttgttaagtatttgctctaggacagagtttgg atttgtttatgtttgctcaaaaggagacccatgggctctc cagggtgcactgagtcaatctagtcctaaaaagcaatctt attattaactctgtatgacagaatcatgtctggaactttt gttttctgctttctgtcaagtataaacttcactttgatgc tgtacttgcaaaatcacattttctttctggaaattccggc agtgtaccttgactgctagctaccctgtgccagaaaagcc tcattcgttgtgcttgaacccttgaatgccaccagctgtc atcactacacagccctcctaagaggcttcctggaggtttc gagattcagatgccctgggagatcccagagtttcctttcc ctcttggccatattctggtgtcaatgacaaggagtacctt ggctttgccacatgtcaaggctgaagaaacagtgtctcca acagagctccttgtgttatctgtttgtacatgtgcatttg tacagtaattggtgtgacagtgttctttgtgtgaattaca ggcaagaattgtggctgagcaaggcacatagtctactcag tctattcctaagtcctaactcctccttgtggtgttggatt tgtaaggcactttatcccttttgtctcatgtttcatcgta aatggcataggcagagatgatacctaattctgcatttgat tgtcactttttgtacctgcattaatttaataaaatattct tatttattttgttacttggtacaccagcatgtccattttc ttgtttattttgtgtttaataaaatgttcagtttaacatc cca | SEQ ID NO: 64 |
| PD-L1 mRNA Full sequence | agttctgcgcagcttcccgaggctccgcaccagccgcgct tctgtccgcctgcagggcattccagaaagatgaggatatt tgctgtctttatattcatgacctactggcatttgctgaac gcatttactgtcacggttcccaaggacctatatgtggtag agtatggtagcaatatgacaattgaatgcaaattcccagt agaaaaacaattagacctggctgcactaattgtctattgg gaaatggaggataagaacattattcaatttgtgcatggag aggaagacctgaaggttcagcatagtagctacagacagag ggcccggctgttgaaggaccagctctccctgggaaatgct gcacttcagatcacagatgtgaaattgcaggatgcagggg tgtaccgctgcatgatcagctatggtggtgccgactacaa gcgaattactgtgaaagtcaatgccccatacaacaaaatc aaccaaagaattttggttgtggatccagtcacctctgaac atgaactgacatgtcaggctgagggctaccccaaggccga agtcatctggacaagcagtgaccatcaagtcctgagtggt aagaccaccaccaccaattccaagagagaggagaagcttt | SEQ ID NO:130 |
| | tcaatgtgaccagcacactgagaatcaacacaacaactaa tgagattttctactgcacttttaggagattagatcctgag gaaaaccatacagctgaattggtcatcccagaactacctc tggcacatcctccaaatgaaaggactcacttggtaattct gggagccatcttattatgccttggtgtagcactgacattc atcttccgtttaagaaaagggagaatgatggatgtgaaaa aatgtggcatccaagatacaaactcaaagaagcaaagtga tacacatttggaggagacgtaatccagcattggaacttct gatcttcaagcagggattctcaacctgtggtttaggggtt catcggggctgagcgtgacaagaggaaggaatgggcccgt gggatgcaggcaatgtgggacttaaaaggcccaagcactg aaaatggaacctggcgaaagcagaggaggagaatgaagaa agatggagtcaaacagggagcctggagggagaccttgata ctttcaaatgcctgaggggctcatcgacgcctgtgacagg gagaaaggatacttctgaacaaggagcctccaagcaaatc atccattgctcatcctaggaagacgggttgagaatcccta atttgagggtcagttcctgcagaagtgccctttgcctcca ctcaatcctcaatttgttttctgcatgactgagagtctca gtgttggaacgggacagtatttatgtatgagtttttccta tttattttgagtctgtgaggtcttcttgtcatgtgagtgt ggttgtgaatgatttcttttgaagatatattgtagtagat gttacaattttgtcgccaaactaaacttgctgcttaatga tttgctcacatctagtaaaacatggagtatttgtaaggtg cttggtctcctctataactacaagtatacattggaagcat aaagatcaaaccgttggttgcataggatgtcacctttatt taacccattaatactctggttgacctaatcttattctcag acctcaagtgtctgtgcagtatctgttccatttaaatatc agctttacaattatgtggtagcctacacacataatctcat ttcatcgctgtaaccaccctgttgtgataaccactattat tttacccatcgtacagctgaggaagcaaacagattaagta acttgcccaaaccagtaaatagcagacctcagactgccac ccactgtccttttataatacaatttacagctatattttac tttaagcaattcttttattcaaaaaccatttattaagtgc ccttgcaatatcaatcgctgtgccaggcattgaatctaca gatgtgagcaagacaaagtacctgtcctcaaggagctcat agtataatgaggagattaacaagaaaatgtattattacaa tttagtccagtgtcatagcataaggatgatgcgaggggaa aacccgagcagtgttgccaagaggaggaaataggccaatg tggtctgggacggttggatatacttaaacatcttaataat cagagtaattttcatttacaaagagaggtcggtacttaaa ataaccctgaaaaataacactggaattccttttctagcat tatatttattcctgatttgcctttgccatataatctaatg cttgtttatatagtgtctggtattgtttaacagttctgtc ttttctatttaaatgccactaaattttaaattcatacctt tccatgattcaaaattcaaaagatcccatgggagatggtt ggaaaatctccacttcatcctccaagccattcaagtttcc tttccagaagcaactgctactgcctttcattcatatgttc ttctaaagatagtctacatttggaaatgtatgttaaaagc acgtatttttaaaatttttttcctaaatagtaacacattg tatgtctgctgtgtactttgctatttttatttattttagt gtttcttatatagcagatggaatgaatttgaagttcccag ggctgaggatccatgccttctttgtttctaagttatcttt cccatagcttttcattatctttcatatgatccagtatatg ttaaatatgtcctacatatacatttagacaaccaccattt gttaagtatttgctctaggacagagtttggatttgtttat gtttgctcaaaaggagacccatgggctctccagggtgcac tgagtcaatctagtcctaaaaagcaatcttattattaact ctgtatgacagaatcatgtctggaacttttgttttctgct ttctgtcaagtataaacttcactttgatgctgtacttgca aaatcacattttctttctggaaattccggcagtgtacctt | |
| | gactgctagctaccctgtgccagaaaagcctcattcgttg tgcttgaacccttgaatgccaccagctgtcatcactacac agccctcctaagaggcttcctggaggtttcgagattcaga tgccctgggagatcccagagtttcctttccctcttggcca tattctggtgtcaatgacaaggagtaccttggctttgcca catgtcaaggctgaagaaacagtgtctccaacagagctcc ttgtgttatctgtttgtacatgtgcatttgtacagtaatt ggtgtgacagtgttctttgtgtgaattacaggcaagaatt gtggctgagcaaggcacatagtctactcagtctattccta agtcctaactcctccttgtggtgttggatttgtaaggcac tttatcccttttgtctcatgtttcatcgtaaatggcatag gcagagatgatacctaattctgcatttgattgtcactttt tgtacctgcattaatttaataaaatattcttatttatttt gttacttggtacaccagcatgtccattttcttgtttattt tgtgtttaataaaatgttcagtttaacatccca | |

In a preferred embodiment, the nucleic acid is capable of reducing gene expression of PD-L1 by gene silencing, preferably wherein the gene silencing by the nucleic acid reduces translation of PD-L1.

In an embodiment, the nucleic acid of the invention targets one, two or three regions, preferably at least two regions, of the PD-L1 transcript selected from the coding region (CDS), the 3' UTR-1 and the 3'UTR-2 of PD-L1 as defined in SEQ ID NOs: 62-64.

In a preferred embodiment, the nucleic acid is capable of hybridizing to both the 3' UTR and the CDS of a PD-L1 mRNA.

In an embodiment, "targeting" or "targets" or other grammatical forms thereof means capable of hybridizing to the CDS, the 3' UTR-1 and/or the 3'UTR-2 of PD-L1.

In an embodiment, the nucleic acid of the invention targets the CDS, the 3' UTR-1 and the 3'UTR-2 of PD-L1 as defined in SEQ ID NOs: 62-64.

In an embodiment the nucleic acid is capable of hybridizing to the 3' UTR (SEQ ID NO: 63 or 64) and comprises a miRNA or a miRNA mimic sequence selected from hsa-miR-103a-3p, hsa-miR-106b-3p, hsa-miR-140-5p, hsa-miR-146b-5p, hsa-miR-151a-3p, hsa-miR-181a-5p, hsa-miR-20a-5p, hsa-miR-222-3p, hsa-miR-23a-3p, hsa-miR-24-3p, hsa-miR-25-3p, hsa-miR-27b-3p, hsa-miR-29b-3p, hsa-miR-3074-5p, hsa-miR-30b-5p, hsa-miR-30c-5p, hsa-miR-30e-5p, hsa-miR-320b, hsa-miR-320c, hsa-miR-340-5p, hsa-miR-425-5p, hsa-miR-4521, hsa-miR-532-3p, hsa-miR-574-3p, hsa-miR-629-5p, and hsa-miR-9-5p.

In an embodiment the nucleic acid is capable of hybridizing to the 3' UTR-1 of PD-L1 (SEQ ID NO: 63) and comprises a miRNA or a miRNA mimic sequence selected from the group consisting of hsa-miR-140-5p, hsa-miR-425-5p, hsa-miR-30b-5p, hsa-miR-574-3p, hsa-miR-29b-3p, hsa-miR-9-5p, hsa-miR-320b, hsa-miR-25-3p, hsa-miR-103a-3p, hsa-miR-320c, hsa-miR-629-5p, hsa-miR-24-3p, hsa-miR-3074-5p, hsa-miR-532-3p, hsa-miR-181a-5p, hsa-miR-27b-3p, hsa-miR-30c-5p, hsa-miR-151a-3p, hsa-miR-4521, hsa-miR-106b-3p and hsa-miR-23a-3p.

In an embodiment the nucleic acid is capable of hybridizing to the 3' UTR-2 of PD-L1 (SEQ ID NO: 64) and comprises a miRNA or a miRNA mimic sequence selected from the group consisting of hsa-miR-20a-5p, hsa-miR-146b-5p, hsa-miR-3074-5p, hsa-miR-24-3p, hsa-miR-151a-3p, hsa-miR-30e-5p, hsa-miR-103a-3p, hsa-miR-222-3p and hsa-miR-340-5p.

In an embodiment the nucleic acid is capable of hybridizing to the CDS of PD-L1 (SEQ ID NO: 62) and comprises a miRNA or a miRNA mimic sequence selected from the group consisting of hsa-miR-584-5p, hsa-miR-9-5p, hsa-miR-550a-3-5p, hsa-miR-550a-5p, hsa-miR-454-3p, hsa-miR-27b-3p, hsa-let-7g-5p, hsa-miR-181a-5p, hsa-miR-122-5p, hsa-miR-3591-3p, hsa-miR-20a-5p, hsa-miR-27a-3p, hsa-miR-103a-3p, hsa-miR-185-3p, hsa-miR-25-5p, hsa-miR-222-3p, , hsa-miR-629-5p, hsa-miR-4521, hsa-let-7e-5p, hsa-miR-211-5p, hsa-miR-221-3p, hsa-let-7f-5p, hsa-miR-128-3p, hsa-miR-93-5p, hsa-let-7i-5p, hsa-let-7c-5p, hsa-miR-23a-3p, hsa-miR-378a-3p, hsa-miR-105-5p, hsa-let-7b-5p, hsa-miR-193a-5p, hsa-miR-451a, hsa-miR-103a-2-5p and hsa-miR-550b-3p.

In an embodiment, the nucleic acid comprises a miRNA or miRNA mimic, wherein the a miRNA or miRNA mimic is capable of targeting PD-L1, wherein the miRNA or miRNA mimic comprises a conserved region at the 5' end of the miRNA or miRNA mimic, in nucleotides 2-7, 3-8, 3-7, 1-7, or 1-8, as counted from the 5' end of the miRNA or miRNA mimic, and wherein the conserved region has a perfect match with the target sequence.

In a preferred embodiment, the nucleic acid targeting the CDS, the 3' UTR-1 and the 3'UTR-2 of PD-L1 comprises a miRNA or a miRNA mimic selected from any one of hsa-miR-103a-3p, hsa-miR-103b, hsa-miR-107, hsa-miR-148a-3p, hsa-miR-148b-3p, hsa-miR-152-3p, hsa-miR-181a-5p, hsa-miR-181b-5p, hsa-miR-211-5p, hsa-miR-23a-3p, hsa-miR-25-5p, hsa-miR-34a-5p, hsa-miR-454-3p, hsa-miR-584-5p and hsa-miR-629-5p as shown in Table 1 above. In a more preferred embodiment, the nucleic acid as defined herein comprises a miRNA or a miRNA mimic selected from any one of hsa-miR-103a-3p, hsa-miR-103b, hsa-miR-148a-3p, hsa-miR-148b-3p, hsa-miR-152-3p, hsa-miR-181a-5p, hsa-miR-181b-5p and hsa-miR-34a-5p.

In an embodiment, the nucleic acid of the invention targets both the CDS and the 3'UTR-2 of PD-L1 as defined in SEQ ID Nos: 62 and 64. In a preferred embodiment, the nucleic acid targeting both the CDS and the 3'UTR-2 of PD-L1 comprises a miRNA or a miRNA mimic selected from hsa-miR-105-5p, hsa-miR-17-5p, hsa-miR-20a-5p, hsa-miR-222-3p, hsa-miR-451a, hsa-miR-486-3p, hsa-miR-486-5p, and hsa-miR-93-5p as shown in Table 1 above. In a preferred embodiment, the nucleic acid as defined above does not include hsa-miR-17-5p.

In a further embodiment, the nucleic acid of the invention targets both the CDS and the 3'UTR-1 of PD-L1 as shown in Table 2 (SEQ ID NOs: 62 and 63). In an embodiment, targeting means capable of hybridizing to the 3'UTR of PD-L1. In an embodiment, the nucleic acid targets the second half (i.e. the downstream half or the 3' half) of the 3'UTR of the mRNA of PD-L1. In a preferred embodiment, the nucleic acid targets the 3'UTR-2 sequence as shown in Table 2 (SEQ ID NO: 64).

In an embodiment, the nucleic acid of the invention targets both the 3'UTR-1 and the 3'UTR-2 of PD-L1 as defined in SEQ ID Nos: 63 and 64. In a preferred embodiment, the nucleic acid targeting both the 3'UTR-1 and the 3'UTR-2 of PD-L1 comprises a miRNA or a miRNA mimic selected from hsa-miR-135b-5p, hsa-miR-140-5p, hsa-miR-151a-3p, hsa-miR-24-3p, hsa-miR-26b-5p, hsa-miR-27b-5p, hsa-miR-3074-5p, hsa-miR-340-5p and hsa-miR-509-3p as shown in Table 1 above. In a preferred embodiment, the nucleic acid as defined above comprises a miRNA or a miRNA mimic selected from hsa-miR-26b-5p and hsa-miR-340-5p.

In an embodiment, the nucleic acid of the invention targets the coding region (CDS) of PD-L1. In an embodiment, "targeting" means capable of hybridizing to the CDS of PD-L1. In a preferred embodiment, the nucleic acid targets the CDS sequence as shown in Table 2 (SEQ ID NO: 62).

In a further embodiment, the nucleic acid of the invention targets both the CDS and the 3'UTR of PD-L1. In an embodiment, targeting means capable of hybridizing to both the CDS and the 3'UTR of PD-L1. In an embodiment, the nucleic acid of the invention comprises a miRNA sequence or a miRNA mimic targeting the 3'UTR of PD-L1, preferably the miRNA or a miRNA mimic targets both the 3'UTR-1 and the 3'UTR-2. In a more preferred embodiment, the nucleic acid of the invention comprises a miRNA sequence or a miRNA mimic targeting the CDS of PD-L1.

In a preferred embodiment, the nucleic acid targeting both the CDS and 3'UTR-1 comprises the sequence of miR-103 and/or the sequence of miR-186-5p as shown in Table 1.

In certain embodiments, the nucleic acid has a first strand, whose sequence is identical to all or part of a mature miRNA sequence, such as the sequences of Table 1, and a second strand or a sense strand whose sequence is about 70% to about 100% (e.g. 75%, 80%, 85%, 90%, or 95%) complementary to the sequence of the first strand.

In one embodiment, the first strand of the nucleic acid is at least about 75%, 80%, 85%, 90%, 95%, or 100% identical, including all integers therein between, to the entire sequence of a mature, naturally occurring miRNA sequence.

In certain embodiments, the first strand is about or is at least about 90%, 91%, 92%, 93%, 94%, 95%, 96%, 97%, 98%, 99%, or 100% identical to the sequence of a mature, naturally-occurring miRNA, such as the mouse, human, or rat miRNA sequence. Alternatively, the first strand may comprise 20, 21, 22, or 23 nucleotide positions in common with a mature, naturally occurring miRNA as compared by sequence alignment algorithms and methods well known in the art.

In some embodiments, the miRNA binding site includes a sequence that has complete complementarity with a miRNA seed sequence of the miRNAs of Table 1. In a preferred embodiment the nucleic acid seed binds to PD-L1 as shown in Table 5.

In certain embodiments, the first strand and the second strand of the nucleic acid comprise ribonucleotides and/or modified ribonucleotides.

In a further embodiment of the invention, the nucleic acid is delivered via liposomes, polymers, conjugates, exosomes, viral vectors, CAR-T cells, or any combinations thereof as further described herein.

In an embodiment, the nucleic acid of the invention is capable of reducing gene expression of PD-L1. In an embodiment, the nucleic acid of the invention is capable of regulating gene expression in a variety of manners, including translational repression, mRNA cleavage, and/or deadenylation, imitating the native miRNA. In a preferred embodiment, the nucleic acid of the invention is capable of inducing PD-L1 downregulation, reducing gene expression of PD-L1 by gene silencing. Preferably, the nucleic acid is capable of reducing gene translation via mRNA cleavage, deadenylation and/or RNA methylation.

In an embodiment the nucleic acid-induced PD-L1 downregulation enhances T cell-mediated cytotoxicity against the cells whose PD-L1 expression has been downregulated. In a preferred embodiment the ratio of activated immune cell to the target cell to which the effector cell is directed (referred herein as effector cell to target cell (E:T) ratio) is of around 10:1 or around 5:1.

In an embodiment, the nucleic acid of the invention is capable of co-regulating PD-L1 expression and other genes of the PD-1/PD-L1 pathway, such as c-FOS, in PD-L1⁺ or PD-L1⁻ cancers. In a preferred embodiment the nucleic acids of the invention that are capable to co-regulate PD-L1 expression in PD-L1⁺ cancers comprise miRNAs miR-186-5P and miR-155-5P, or miR-186-5P and miR-181b-5P, both of which show a strong correlation/co-regulation effect in PD-L1⁺ patients. In an equally preferred embodiment the nucleic acids of the invention that are able to co-regulate PD-L1 expression in PD-L1⁺ cancers comprise the miRNA sequence of miR-181b-5P which have a strong association with all investigated miRNAs pairs of miRNAs. In an equally preferred embodiment the nucleic acids of the invention that are able to co-regulate PD-L1 expression in PD-L1⁻ cancers comprise the pairs of miRNAs sequences selected from miR-29a and miR-17, which were more strongly correlated in PD-L1⁻ patients.

The skilled person will appreciate that the nucleic acid of the invention has a particular length. The length of the nucleic acid is chosen, so as to enable the skilled person to silence the expression of PD-L1. In particular, the nucleic acid may have a length of from 17 to 1000 nucleotides. The nucleic acid may be from 17 to 950, from 17 to 900, from 17 to 850, from 17 to 800, from 17 to 750, from 17 to 700, from 17 to 650, from 17 to 600, from 17 to 550, from 17 to 500, from 17 to 450, from 17 to 400, from 17 to 350, from 17 to 300, from 17 to 250, from 17 to 200, from 17 to 150, from 17 to 120, from 17 to 110, from 17 to 100, from 17 to 90, from 17 to 80, from 17 to 70, from 17 to 60, from 17 to 50, from 17 to 40, from 17 to 30, from 17 to 27, from 17 to 26, from 17 to 25, from 17 to 24, from 17 to 23, from 17 to 22, from 17 to 21, from 17 to 20, from 17 to 19, or from 17 to 18 nucleotides long. Particularly preferred are lengths that are sufficient for delivering the nucleic acid into a target cell, for instance by transfection.

The nucleic acid may be comprised in a vector. The vector can be used to deliver the nucleic acid into a target cell, as e.g. described elsewhere herein. The vector may be a plasmid or a viral vector, such as a lentiviral or an adenoviral vector. The skilled person is aware of vectors for the delivery of nucleic acids to target cells and organisms, e.g. human. In particular, the skilled person knows such vectors for delivering nucleic acids comprising miRNAs or miRNA mimics.

In some embodiments a cell may be a target cell as taught herein. In a preferred embodiment, the target cell is a cancer cell, for instance a cancer cell of a cancer to be treated as taught herein.

### miRNA mimics

In a further aspect, the invention provides a miRNA mimic comprising a sequence that has at least 70%, at least 75%, at least 80%, at least 85%, at least 90%, at least 95% or 100% sequence identity with a sequence selected from the group consisting of the sequences according to any one of SEQ ID NOs: 1 to 60.

In a preferred embodiment the miRNA mimic is capable of hybridizing to a PD-L1 mRNA, and/or the miRNA mimic is capable of reducing gene expression of PD-L1, more preferably the miRNA mimic reduces translation of the PD-L1 mRNA.

In a preferred embodiment, the miRNA mimic comprises a sequence selected from the group consisting of the sequences according to any one of SEQ ID NOs: 1 to 60.

In a preferred embodiment, the nucleic acid of the invention is a miRNA mimic. Preparing the nucleic acid of the invention as a miRNA mimic increases the stability of the nucleic acid towards degradation, in particular degradation by nucleases.

In an embodiment, the miRNA mimic comprises any one of the sequences of Table 1.

In an embodiment, the miRNA mimic of the invention targets the CDS, the 3' UTR-1 and the 3'UTR-2 of PD-L1 as defined in SEQ ID Nos: 62-64. In a preferred embodiment, the miRNA mimic targeting all three regions comprises a miRNA sequence selected from any one of hsa-miR-103a-3p, hsa-miR-103b, hsa-miR-107, hsa-miR-148a-3p, hsa-miR-148b-3p, hsa-miR-152-3p, hsa-miR-181a-5p, hsa-miR-181b-5p, hsa-miR-211-5p, hsa-miR-23a-3p, hsa-miR-25-5p, hsa-miR-34a-5p, hsa-miR-454-3p, hsa-miR-584-5p, and/or hsa-miR-629-5p as shown in Table 1 above. In a more preferred embodiment, the miRNA mimic as defined above comprises a miRNA sequence selected from any one of hsa-miR-103a-3p, hsa-miR-103b, hsa-miR-148a-3p, hsa-miR-148b-3p, hsa-miR-152-3p, hsa-miR-181a-5p, hsa-miR-181b-5p and hsa-miR-34a-5p.

In an embodiment, the miRNA mimic of the invention targets both the CDS and the 3'UTR-2 of PD-L1 as defined in SEQ ID Nos: 62 and 64. In a preferred embodiment, the miRNA mimic targeting both the CDS and the 3'UTR-2 of PD-L1 comprises a miRNA sequence selected from hsa-miR-105-5p, hsa-miR-17-5p, hsa-miR-20a-5p, hsa-miR-222-3p, hsa-miR-451a, hsa-miR-486-3p, hsa-miR-486-5p, and/or hsa-miR-93-5p as shown in Table 1 above. In a preferred embodiment, the miRNA mimic as defined above does not include hsa-miR-17-5p.

In a further embodiment, the miRNA mimic of the invention targets the 3'UTR of PD-L1 as shown in Table 2 (SEQ ID NOs: 62 and 63). In an embodiment, targeting means capable of hybridizing to the 3'UTR of PD-L1. As disclosed herein, the strongest effects in the reduction of PD-L1 expression is achieved when the miRNA mimic targets the 3'UTR of PD-L1. In an embodiment, the miRNA mimic targets the second half (i.e. the downstream half or the 3' half) of the 3'UTR of the mRNA of PD-L1. In a preferred embodiment, the miRNA mimic targets the 3'UTR-2 sequence as shown in Table 2 (SEQ ID NO: 64).

In an embodiment, the miRNA mimic of the invention targets both the 3'UTR-1 and the 3'UTR-2 of PD-L1 as defined in SEQ ID Nos: 63 and 64. In a preferred embodiment, the miRNA mimic targeting both the 3'UTR-1 and the 3'UTR-2 of PD-L1 is a miRNA selected from hsa-miR-135b-5p, hsa-miR-140-5p, hsa-miR-151a-3p, hsa-miR-24-3p, hsa-miR-26b-5p, hsa-miR-27b-5p, hsa-miR-3074-5p, hsa-miR-340-5p and hsa-miR-509-3p as shown in Table 1 above. In a preferred embodiment, the miRNA mimic as defined above is a miRNA selected from hsa-miR-26b-5p and hsa-miR-340-5p.

In a preferred embodiment, the miRNA mimic comprises the sequence of hsa-mir-26a-5p (SEQ ID NO: 1), hsa-miR-26b-5p (SEQ ID NO: 2), hsa-miR-29a-3p, hsa-miR-103b (SEQ ID NO: 4), hsa-miR-181b-5p (SEQ ID NO: 5) or hsa-miR-186-5p (SEQ ID NO: 6). In a more preferred embodiment, the miRNA mimic consists of the sequence of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) or hsa-miR-186-5p (SEQ ID NO: 6).

In certain embodiments, the miRNA mimics have a first strand, whose sequence is identical to all or part of a mature miRNA sequence, such as the sequences of Table 1, and a second strand or a sense strand whose sequence is about 70% to about 100% (e.g. 75%, 80%, 85%, 90%, or 95%) complementary to the sequence of the first strand.

In one embodiment, the first strand of the miRNA mimic is at least about 75%, 80%, 85%, 90%, 95%, or 100% identical, including all integers therein between, to the entire sequence of a mature, naturally occurring miRNA sequence.

In certain embodiments, the first strand is about or is at least about 90%, 91%, 92%, 93%, 94%, 95%, 96%, 97%, 98%, 99%, or 100% identical to the sequence of a mature, naturally-occurring miRNA, such as the mouse, human, or rat miRNA sequence. Alternatively, the first strand may comprise 20, 21, 22, or 23 nucleotide positions in common with a mature, naturally occurring miRNA as compared by sequence alignment algorithms and methods well known in the art.

In some embodiments, the miRNA mimic comprises a sequence that has complete complementarity with a miRNA seed sequence of the miRNAs of Table 1. In a preferred embodiment the miRNA mimic comprises a seed sequence that binds to PD-L1 as shown in Table 5. In certain embodiments, the first strand and the second strand of the microRNA mimic comprise ribonucleotides and/or modified ribonucleotides.

In some embodiments, the double stranded microRNA mimic of the invention comprises a first strand and a second strand, wherein the first strand comprises at least about 24 ribonucleotides and the second strand comprises at least about 22 ribonucleotides, wherein the second strand comprises a sequence that is substantially complementary to the first strand, and wherein the second strand comprises at least one stretch of three consecutive ribonucleotides comprising a 2'-fluoro-ribonucleotide.

In some embodiments, a double stranded microRNA mimic of the invention comprises:
(a) a first strand comprising about 21 nucleotides, and (b) a second strand comprising at least about 23 nucleotides, wherein the first strand comprises about 4 to about 9 deoxyribonucleotides and about 16 to about 21 ribonucleotides, and wherein the second strand comprises about 6 to about 7 deoxyribonucleotides and about 16 to about 17 ribonucleotides.

It is understood that the sequence of the first strand is considered to be identical to the sequence of a mature miRNA even if the first strand includes a modified nucleotide instead of a naturally-occurring nucleotide. For example, if a naturally occurring miRNA sequence comprises a cytidine nucleotide at a specific position, the first strand of the miRNA mimic may comprise a modified cytidine nucleotide, such as 2'-fluoro-cytidine, at the corresponding position or if a naturally occurring miRNA comprises a uridine nucleotide at a specific position, the miRNA mimic may comprise a modified uridine nucleotide, such as 2'-fluoro-uridine, 2'-O-methyl-uridine, 5-fluorouracil, or 4-thiouracil at the corresponding position. Thus, as long as the modified nucleotide has the same base-pairing capability as the nucleotide present in the naturally occurring miRNA, the sequence of the first strand is considered to be identical to the naturally-occurring miRNA sequence. In some embodiments, the first strand may include a modification of the 5'-terminal residue. For example, the first strand may have a 5'-terminal monophosphate. In some other embodiments, the first strand does not contain a 5'-terminal monophosphate.

In some embodiments, the second strand of the microRNA mimic is partially complementary to the sequence of the first strand. For example, the sequence of the second strand is at least about 70%, 75%, 80%, 85%, 90%, 95%, 96%, 97%, 98%, or 99%, inclusive of all values there between, complementary to the sequence of the first strand. In some other embodiments, the second strand is substantially complementary to the sequence of the first strand. For example, the second strand is at least about 85%, 86%, 87%, 88%, 89%, 90%, 91%, 92%, 93%, 94%, 95%, 96%, 97%, 98%, or 99%, inclusive of all values therebetween, complementary to the sequence of the first strand. In yet some other embodiments, the sequence of the second strand may be fully complementary to the first strand. In certain embodiments, about 19, 20, 21, 22, or 23 nucleotides of the complementary region of the second strand may be complementary to the first strand.

It is understood that the sequence of the second strand is considered complementary to the first strand even if the second strand includes a modified nucleotide instead of a naturally occurring nucleotide. For example, if the first strand sequence comprises a guanosine nucleotide at a specific position, the second strand may comprise a modified cytidine nucleotide, such as 2'-O-methyl-cytidine, at the corresponding position.

In some embodiments, the second strand comprises about 1, 2, 3, 4, 5, or 6 mismatches relative to the first strand. That is, up to 1, 2, 3, 4, 5, or 6 nucleotides between the first strand and the second strand may not be complementary. In one embodiment, the mismatches are not consecutive and are distributed throughout the second strand. In another embodiment, the mismatches are consecutive and may create a bulge.

In some embodiments, the first and/or the second strand of the mimic may comprise an overhang on the 5' or 3' end of the strands. In certain embodiments, the first strand comprises a 3' overhang, i.e., a single-stranded region that extends beyond the duplex region, relative to the second strand. The 3' overhang of the first strand may range from about one nucleotide to about four nucleotides. In certain embodiments, the 3' overhang of the first strand may comprise 1 or 2 nucleotides. In some embodiments, the nucleotides comprising the 3' overhang in the first strand are linked by phosphorothioate linkages. The nucleotides comprising the 3' overhang in the first strand may include ribonucleotides, deoxyribonucleotides, modified nucleotides, or combinations thereof. In certain embodiments, the 3' overhang in the first strand comprises two ribonucleotides. In one embodiment, the 3' overhang of the first strand comprises two uridine nucleotides linked through a phosphorothioate linkage. In some embodiments, the first strand may not contain an overhang.

In some embodiments, the nucleotides in the second strand of miRNA mimics of the disclosure are linked by phosphodiester linkages and the nucleotides in the first strand are linked by phosphodiester linkages except for the last three nucleotides at the 3' end which are linked to each other via phosphorothioate linkages.

The first strand of the miRNA mimic may comprises one or more 2'-fluoro modified nucleotides. In another embodiment, the first strand may not include any modified nucleotide. In one embodiment, the second strand comprises one or more 2'-O-methyl modified nucleotides. The modified nucleotides that may be used in the miRNA mimics of the disclosure can include nucleotides with a base modification or substitution. The natural or unmodified bases in RNA are the purine bases adenine (A) and guanine (G), and the pyrimidine bases cytosine (C) and uracil (U) (DNA has thymine (T)). In contrast, modified bases, such as heterocyclic base moieties, include other synthetic and natural nucleobases, such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8- thioalkyl, 8-hydroxyl and other 8-substituted adenines and guanines, 5-halo (including 5-bromo, 5-trifluoromethyl and other 5-substituted uracils and cytosines), 7-methylguanine and 7- methyladenine, 2-F-adenine, 2-aminoadenine, 8-azaguanine and 8-azaadenine, 7-deazaguanine and 7-deazaadenine and 3-deazaguanine and 3-deazaadenine.

In some embodiments, the miRNA mimic comprises nucleotides with modified sugar moieties. Representative modified sugars include carbocyclic or acyclic sugars, sugars having substituent groups at one or more of their 2', 3 ' or 4 ' positions and sugars having substituents in place of one or more hydrogen atoms of the sugar. In certain embodiments, the sugar is modified by having a substituent group at the 2' position. In additional embodiments, the sugar is modified by having a substituent group at the 3' position. In other embodiments, the sugar is modified by having a substituent group at the 4' position. It is also contemplated that a sugar may have a modification at more than one of those positions, or that an RNA molecule may have one or more nucleotides with a sugar modification at one position and also one or more nucleotides with a sugar modification at a different position.

Sugar modifications contemplated in the miRNA mimic include, but are not limited to, a substituent group selected from: OH, F, O-, S-, or N-alkyl; O-, S-, or N-alkenyl; O-, S- or N-alkynyl; O-alkyl-O-alkyl, wherein the alkyl, alkenyl and alkynyl may be substituted or unsubstituted C2 to C10 alkenyl and alkynyl.

In some embodiments, the miRNA mimic has a sugar substituent group selected from the following: C2 to C10 lower alkyl, substituted lower alkyl, alkenyl, alkynyl, alkaryl, aralkyl, O-alkaryl or O-aralkyl, SH, SCH₃, Cl, Br, CN, OCN, CF₃, OCF₃, SOCH₃, SO₂CH₃, ONO₂, NO₂, N₃, NH₂, heterocycloalkyl, heterocycloalkaryl, aminoalkylamino, or similar substituents. In one embodiment, the modification includes 2'-methoxyethoxy (2'-O-CH₂CH₂OCH₃, which is also known as 2'-O-(2-methoxyethyl) or 2'-MOE), that is, an alkoxyalkoxy group. Another modification includes 2'-dimethylaminooxyethoxy (2'-O(CH₂)₂ON(CH₃)₂) and 2'-dimethylaminoethoxyethoxy 2'-O(CH₂)₂O(CH₂)₂N(CH₃)₂.

Sugar substituent groups on the 2' position (2'-) may be in the arabino (up) position or ribo (down) position. One 2'-arabino modification is 2'-F. Other similar modifications may also be made at other positions on the sugar moiety, particularly the 3' position of the sugar on the 3' terminal nucleoside or in 2'-5' linked oligonucleotides and the 5' position of 5' terminal nucleotide.

The first and the second strand of the microRNA mimic can also include backbone modifications, such as one or more phosphorothioate, phosphorodithioate, phosphotriester, boranophosphate, alkylphosphonates, phosphoramidates, phosphordiamidates, thionophosphoramidates, thionoalkylphosphonates, thionoalkylphosphotriesters, or phosphonocarboxylate linkages, where the linkage is the normal 3'-5' linkage, 2'-5' linked analog or inverted linkages such as 3'-3', 5'-5' and 2'-2'. The first and/or the second strand of the microRNA mimic can also include modifications on the sugar residue, such as ribose modification/ replacement. For instance, the first and/or the second strand of the microRNA mimic may include one or more morpholino, peptide nucleic acids, serinol nucleic acids, locked nucleic acids (LNA), and unlocked nucleic acids.

In some embodiments, the microRNA mimic is conjugated to an agent such as a steroid (cholesterol), a vitamin, a fatty acid, a carbohydrate or glycoside, a peptide, or other small molecule ligand to facilitate targeting, delivery, and/or stability. In some embodiments, the agent is attached to the first strand or second strand of the microRNA mimic at its 3' or 5' end through a linker or a spacer group. In some embodiments, the agent is cholesterol, a cholesterol derivative, cholic acid or a cholic acid derivative.

In an embodiment, the miRNA mimic targets both the CDS and the 3'UTR of PD-L1. In a further embodiment, the miRNA mimic targets at least two of the CDS, the 3'UTR-1 and/or the 3'UTR-2 of PD-L1 as shown in Table 2 above. In a further embodiment, the miRNA mimic targets both the 3'UTR-1 and/or the 3'UTR-2 of PD-L1. In a further embodiment, the miRNA targets the 3'UTR of PD-L1. In an embodiment, the miRNA mimic targets specifically the CDS of PD-L1. In a further embodiment, the miRNA targets the 3'UTR of PD-L1. In a preferred embodiment, the miRNA mimic targets the 3'UTR. In a more preferred embodiment, the miRNA targets the second half (i.e the downstream or the 3' half) of the 3'UTR of the mRNA of PD-L1.

In an embodiment, the microRNA reduces gene expression of PD-L1. In an embodiment, the microRNA mimics of the invention regulate gene expression in a variety of manners, including translational repression, mRNA cleavage, and/or deadenylation, imitating the native miRNA. In a preferred embodiment, the miRNA mimic is capable of reducing gene expression of PD-L1 by gene silencing. Preferably, the micro RNA reduces gene translation via mRNA cleavage, deadenylation and/or RNA methylation.

In an embodiment the miRNA mimic-induced PD-L1 downregulation enhances T cell-mediated cytotoxicity against the target cell, in which PD-L1 expression is downregulated. In a preferred embodiment the effector cell - target cell (E:T) ratio is of around 10:1.

### Combinations

In some embodiments combinations of the nucleic acids, the miRNAs, the miRNA mimics or the cells are preferred. Particularly preferred are combinations of the sequences according to any one of SEQ ID NOs: 1-6, e.g. 1 and 2, 1 and 3, 1 and 4, 1 and 5, 1 and 6, 2 and 3, 2 and 4, 2 and 5, 2 and 6, 3 and 4, 3 and 5, 3 and 6, 4 and 5, 4 and 6, or 5 and 6 (all numerals referring to SEQ ID NOs). Further, in some embodiments, any one of SEQ ID NOs: 1-6 may be combined with SEQ ID NO: 7 or SEQ ID NO: 8, or both, e.g. SEQ ID NO:1 and SEQ ID NO: 7 and SEQ ID NO: 8. For instance, the therapeutic methods taught herein may include the administration of two, three, four, five, six or more miRNAs according to the present disclosure, e.g. in the form of a miRNA mimic or in the form of a vector. Particularly preferred combinations are taught elsewhere herein.

### Delivery systems and Vectors

A common problem with nucleic acids as miRNA therapeutics is to maintain the stability and consistency of microRNAs in circulation, since "naked" and 2'OH unmodified microRNAs are quickly degraded by extracellular RNAases. Different solutions to prevent miRNA degradation are known and can be used with the nucleic acids of the present invention.

In a preferred embodiment, in order to avoid miRNA degradation, the miRNA is chemically modified as described herein, for instance with modifications on the phosphodiester backbone and the 2' of the ribose protecting the miRNA from degradation. Different chemical modifications have been developed, such as modified phosphodiester linkages, a modified ribose backbone, 2'-O-(2-Methoxyethyl), 2'-O-Methyl, 2'-locked nucleic acid, and 2'-Fluoro modifications. These and other modifications taught herein improve nucleic acid stability and may additionally increase binding affinity to the target and help loading into the miRNA-induced silencing complex, both of which improve miRNA performance. Modifications typically are selected to be compatible with the various protein components that together produce an active RNA-induced silencing complex (RISC complex). More specifically, in a preferred embodiment the nucleic acid, the miRNA or the miRNA mimic is bound by the RISC complex, followed by removal of the sense strand to produce an active RISC complex.

The nucleic acid, miRNA or miRNA mimic may be delivered to a cell or patient by lipid based nanoparticles as delivery system, for instance when used in the methods and uses disclosed herein. Lipid based nanoparticles are a general group including liposomes, solid lipid nano particles (SLNs), and nanostructured lipid carriers (NLCs). The nucleic acids of the invention may be formulated with polymers for delivery. Polymers may be cationic (e.g. PEI) or neutral (e.g. PLGA) polymers. Highly efficient encapsulation and a net cationic charge allows interaction with anionic miRNAs. Neutral polymers may be coated with cationic reagent for efficient delivery. Examples of polymer based transfection systems are those comprising linear polyethylamine (PEI) derivatives, wherein the nucleic acid is condensed into positively charged complexes, which enter the cell by endocytosis (such as Transporter^{™} 5 Transfection Reagent by Polysciences Inc. (Warrington, PA, US).

In a further embodiment, the nucleic acid, the miRNA or the miRNA mimic of the invention is delivered in the form of conjugates, comprising a lipid or receptor binding molecule, such as an aptamer, bound, preferably covalently bound, to the nucleic acid, the miRNA or the miRNA mimic. Conjugates have the advantage of selective targeting, improved intracellular delivery, high stability and low toxicity.

In a further embodiment, the nucleic acid, the miRNA or the miRNA mimic is delivered via exosomes. Exosomes have the advantage of being small and naturally existing in humans and are able to fuse with the cell membrane and can avoid the endosome. Naturally present in body fluids, are immunocompatible, have low toxicity, and are able to cross the blood brain barrier.

In a further aspect, a vector encodes the nucleic acid. In a preferred embodiment, the vector encodes a nucleic acid comprising any one of SEQ ID NOs: 1-60. More preferably, the vector encodes one or more of the nucleic acids selected from SEQ ID Nos: 1-6. In an embodiment, the vector does not encode miR17 and/or miR155. In a preferred embodiment, the viral vector encodes at least two of the sequences of SEQ ID Nos: 1-60. More preferably, the vector encodes one or more of the nucleic acids selected from SEQ ID Nos: 1-6.

In a further embodiment, the nucleic acid or the miRNA is delivered via viral vectors. Lentiviruses, adenoviruses and adenoassociated viruses (AAVs) can be used to deliver nucleic acids or miRNAs into cells, in particular mammalian cells, such as human cells. Vectors deliver the nucleic acid or miRNA to express miRNAs, pri-miRNAs, pre-miRNAs or mature miRNAs. Viral vectors have high transfection efficiency and low toxicity.

### Cells

In a further aspect, the invention relates to a cell comprising the nucleic acid described herein, the vector described herein or the miRNA mimic described herein. In a preferred embodiment the cell is an immune cell, preferably a CAR T cell.

In an embodiment, the cell comprises the nucleic acid, the miRNA, the vector and/or the miRNA mimic described herein. In an embodiment, the nucleic acid, the miRNA or the miRNA mimic of the invention is delivered in the form of cell-to-cell nucleic acid transfer. In cell-to-cell nucleic acid transfer a carrier cell carrying a nucleic acid is put in contact with or specifically directed to a target cell, transferring the nucleic acids from the carrier to the target cell. In a preferred embodiment, the carrier cell is an immune cell. In a more preferred embodiment the immune cell is a chimeric antigen receptor (CAR) T cell. Preferably, the CAR T cell is directed to an antigen present or presented on the cancer cell surface. In an embodiment the carrier cell, preferably the CAR T cell comprises a bifunctional adapter molecule termed target module (TM) that is able to cross-link CAR T cells with target cells. In an embodiment the nucleic acid transfer from the carrier cell, such as the immune cell or the CAR T, to the target cell occurs via exosomes, macrovesicles or lipid based nanoparticles, such as liposomes, solid lipid nano particles (SLNs), and nanostructured lipid carriers (NLCs). In an embodiment the target cell is a cancer cell. Preferably the cancer is a PD-L1 expressing cancer. In one embodiment the cancer is a skin cancer (e.g. melanoma), non-small cell lung cancer (NSCLC), Hodgkin's lymphoma, bladder cancer, renal cell carcinoma (RCC), head and neck squamous cell carcinoma (HNSCC), breast cancer, Merkel cell carcinoma, hepatocellular carcinoma (HCC) and gastric cancer (GC). In a preferred embodiment, the cancer is skin cancer, e.g. melanoma, non-small cell lung cancer (NSCLC), renal cell carcinoma (RCC), bladder cancer and head and neck squamous cell carcinoma (HNSCC). In a preferred embodiment, the cancer is skin cancer. In a more preferred embodiment, the skin cancer is a melanoma.

In an embodiment the CAR T cell is modified to be switched on and off rapidly and reversibly using technologies known to the skilled person. Preferably, the switching technology comprises the UniCAR system as described in Cartellieri, M., et al. (Blood cancer journal 6.8 (2016): e458-e458), Mitwasi, N., et al. (Scientific reports 10.1 (2020): 1-16) and Arndt, C., et al., (Oncoimmunology 8.11 (2019): 1659095), all of which are incorporated herein by reference for all purposes.

### Therapy

A further aspect of the invention relates to the nucleic acid, miRNA, vector or the miRNA for use in therapy.

In one embodiment, the nucleic acid, the miRNA or the miRNA mimic is for use in treating cancer. In one embodiment, the cancer is a cancer expressing PD-L1 (PD-L1⁺ cancer). In one embodiment the cancer is a skin cancer (e.g. melanoma), non-small cell lung cancer (NSCLC), Hodgkin's lymphoma, bladder cancer, renal cell carcinoma (RCC), head and neck squamous cell carcinoma (HNSCC), breast cancer, Merkel cell carcinoma, hepatocellular carcinoma (HCC) and gastric cancer (GC). In a preferred embodiment, the cancer is melanoma, non-small cell lung cancer (NSCLC), renal cell carcinoma (RCC), bladder cancer and head and neck squamous cell carcinoma (HNSCC). In a preferred embodiment, the cancer is skin cancer. In a more preferred embodiment, the skin cancer is a melanoma.

The nucleic acid, the miRNA, the vector or the miRNA mimic also find use in a method of treating a subject having a cancer, the method comprising the step of administering to the subject an effective amount of the nucleic acid, the vector, miRNA mimic or the miRNA of the invention, thereby treating the cancer.

In an embodiment, the method comprises administering to the subject an effective amount of one or more nucleic acids, miRNAs, miRNA mimics, or vectors comprising a sequence selected from any one of SEQ ID NOs 1-60. In an embodiment the sequences of SEQ ID Nos: 7 and 8 (miR17 and miR155) are excluded. In an embodiment, the sequences of hsa-miR-148a-3p and/or hsa-miR-148b-3p are excluded.

In a preferred embodiment, the method comprises administering to the subject an effective amount of one or more nucleic acids, miRNAs, miRNA mimics, or vectors comprising a sequence selected from the list consisting of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6).

In an embodiment two or more of the nucleic acids, miRNAs, miRNA mimics, or vectors comprising any of the sequences described herein are administered simultaneously or within 1, 2, 3, 4, 5, 7, 8, 9, 10, 11, 12, 13, 14, or 15 days of one other, preferably in amounts sufficient to treat a cancer.

In an embodiment, hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6) are administered in a combination therapy.

In a preferred embodiment, hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and/or hsa-miR-186-5p (SEQ ID NO: 6) are administered in combination with any know miRNA targeting PD-L1 such as miR17 or mir155. In a particularly preferred embodiment hsa-miR-181b-5p (SEQ ID NO: 5) is administered in combination with miR155-5p (SEQ ID NO: 7).

Herein the cancer may be a skin cancer (e.g. melanoma), non-small cell lung cancer (NSCLC), Hodgkin's lymphoma, bladder cancer, renal cell carcinoma (RCC), head and neck squamous cell carcinoma (HNSCC), breast cancer, Merkel cell carcinoma, hepatocellular carcinoma (HCC) and gastric cancer (GC). In a preferred embodiment, the cancer is skin cancer, melanoma, non-small cell lung cancer (NSCLC), renal cell carcinoma (RCC), bladder cancer and head and neck squamous cell carcinoma (HNSCC). In a preferred embodiment, the cancer is skin cancer. The skin cancer preferably is melanoma.

In a further aspect of the invention, the nucleic acid, vector, miRNA or miRNA mimic of the invention is for use in silencing PD-L1 gene expression.

In a further aspect, the invention relates to a pharmaceutical composition comprising the microRNA mimic, the vector and/or the cell described herein.

In a further aspect, the invention relates to a method of treating a subject having a cancer, the method comprising administering to the subject an effective amount of the nucleic acid of the invention as described herein, wherein the nucleic acid reduces expression of PD-L1, thereby treating the cancer.

In a preferred embodiment, the nucleic acid comprises at least one microRNA selected from the group consisting of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6).

In an embodiment, the nucleic acid capable of targeting both 3'UTR-1 and 3'-UTR-2 is capable of causing an equal inhibition of PD-L1 expression, when targeting each 3'UTR RNA region independently. In a preferred embodiment, the reduction in PD-L1 expression is at least 10%, at least 20%, at least 30%, at least 40%, at least 50%, at least 60%, at least 70%, at least 80% or at least 90%, relative to the PD-L1 expression in the absence of the nucleic acid, miRNA or miRNA mimic. In a preferred embodiment, the nucleic acid comprises miRNA miR-181b. As disclosed herein, miR181b expression or overexpression leads to an equal signal reduction of approximately ≈30% in both 3'-UTR segments.

In a preferred embodiment, the nucleic acid comprises a microRNA selected from miR-103b (SEQ ID NO: 4) and wherein the mature miRNA targets the CDS gene region of PD-L1.

In a more preferred embodiment, the nucleic acid comprises miR-155 and/or miR181b-5p, wherein the mature miRNA targets the 3'UTR of PD-L1. In an even more preferred embodiment, miRNA targets the second half downstream of the 3'UTR of PD-L1.

In an embodiment the nucleic acid is capable of causing an equal PD-L1 expression reduction when targeting both the 3'-UTR1 and 3' UTR2 segments of the PD-L1 mRNA. In a preferred embodiment the reduction in PD-L1 expression is at least 10%, at least 20 or at least 30%. In a preferred embodiment the reduction in PD-L1 expression is of approximately 30% in both 3'-UTR1 and 3' UTR2 independently. In a preferred embodiment, the nucleic acid comprises a sequence that has complete complementarity with the miRNA seed sequence of miR181b-5P. In a more preferred embodiment, the nucleic acid comprises miR181b-5P.

### Pharmaceutical compositions

The present disclosure also provides pharmaceutical compositions comprising a therapeutically effective amount of one or more of the nucleic acids, the miRNAs, the miRNA mimics or the vectors according to the disclosure, or a pharmaceutically acceptable salt thereof, and a pharmaceutically acceptable carrier or excipient. In some embodiments, the pharmaceutical compositions of the disclosure comprise a therapeutically effective amount of two or more, e.g., 2, 3, 4, 5, 6, 7, 8, 9, 10, of the nucleic acids, the miRNAs, the miRNA mimics or the vectors of the disclosure and a pharmaceutically acceptable carrier or excipient. In some embodiments, the pharmaceutical composition comprises a pool of the nucleic acids, the miRNAs, the miRNA mimics or the vectors.

### Routes of administration

The disclosure also encompasses embodiments where an additional therapeutic agent may be administered together with the nucleic acid, the miRNA, the miRNA mimic or the vector of the invention. The additional therapeutic agent may be formulated in the same pharmaceutical composition as the nucleic acid, the miRNA, the miRNA mimic or the vector of the invention. The additional therapeutic agent may be administered concurrently, but in a separate formulation or sequentially with the nucleic acid, the miRNA, the miRNA mimic or the vector of the invention. In other embodiments, the additional therapeutic agent may be administered at different times prior to or after administration of the nucleic acid, the miRNA, the miRNA mimic or the vector of the invention.

The nucleic acid, the miRNA, the miRNA mimic or the vector, or the pharmaceutical compositions thereof may be administered via one or more of the following routes of administration: intravenous, intraocular, intravitreal, intramuscular, subcutaneous, topical, oral, transdermal, intraperitoneal, intraorbital, by implantation, by inhalation, intrathecal, intraventricular, via the ear, or intranasal.

### Kits and articles of manufacture

The present disclosure provides kits comprising any one or more of the nucleic acid, the miRNA, the miRNA mimic, the vector and the pharmaceutical composition described herein. The kit may further comprise an instruction leaflet, product insert, or information and directions for use in accordance with the technical teachings herein.

### Regulation of Gene Expression

The nucleic acids, miRNAs, vectors and miRNA mimics of the invention are also useful for regulating the expression of PD-L1 and other genes of thePD-1/PD-L1 pathway, e.g c-Fos. In some embodiments, the present disclosure provides methods of regulating at least one gene in a cell comprising contacting the cell with one or more of the nucleic acids, miRNAs, vectors and miRNA mimics of the invention.

### miRNA expression systems

Nucleic acid and miRNA expression systems can use the cellular pathway for miRNA expression and processing.

Without being bound by theory, it is appreciated that typically miRNAs are generated and processed endogenously as follows. The small, single-stranded miRNAs are initially transcribed as longer primary transcripts of about 60-120 nucleotides in length (termed primary microRNAs (pri-miRNAs)), containing an RNA hairpin, in which one of the two strands includes the mature miRNA. The hairpin containing pri-miRNAs are cleaved in two steps by two RNase III enzymes Drosha and Dicer. In a first step in the nucleus, Drosha yields an approximately 70 nucleotide long precursor miRNA (called pre-miRNA). The pre-miRNAs are transported to the cytoplasm via Exportin-5 and further processed by Dicer to produce a short (about 20-25 nucleotide long) partially double-stranded RNA (usually 22 nt long), in which one strand is the mature miRNA. The methods and uses of the invention may exploit this intracellular machinery when administering the nucleic acid, miRNA, vector or miRNA mimic to cells.

In an embodiment of the invention, upregulation or overexpression of the nucleic acid of the invention can be accomplished by using chemically synthesized miRNA mimics, or shRNA-like or intronic miRNA expression vectors to express the primary miRNA or pre-miRNA. miRNA expression can thus be based on the transfection and expression in the nucleus of the pri-miRNA, or the transfection and expression in the cytoplasm of pre-miRNA and/or the expression of double stranded miRNAs or finally expression/transfection of mature miRNAs.

The different aspects and embodiments of the invention can use miRNA expression vectors for the efficient expression of the nucleic acids or miRNAs of the invention. Conventional pri-miRNA expression vectors comprise expression cassettes with a strong promotor, such as CMV promoter in combination with terminator sequence, such as SV40 Poly(A). The pri-miRNA follows the cellular miRNA production machinery, and is processed by Drosha and dicer to generate a mature miRNA that is loaded onto the RISC complex.

In a further preferred embodiment, the pre-miRNA can be expressed using a strong promoter such as U6, thereby avoiding the Drosha RNAse III machinery.

In a further preferred embodiment, one or more of the nucleic acids or miRNAs of the present invention can be directly expressed as duplexes of small RNAs using oligo expression cassettes. In these vectors, the expression of mature miRNAs is driven by convergent promoters in opposing directions, respectively, such as U6 and H1, with respective transcriptional termination signals (such as TTTTT or UUUUU) inserted between the mature miRNAs.

### Therapeutic Indications

The present disclosure provides methods of treating, ameliorating, or preventing one or more conditions in a subject in need thereof comprising administering to the subject a therapeutically effective amount of at least one of the nucleic acids or the miRNA mimics described herein.

In an embodiment, the nucleic acids or the miRNA mimics of the invention are used in a method of treatment of cancer, preferably skin cancer and more preferably melanoma.

### EXAMPLES

The following examples are provided for purposes of illustration only and are not intended to be limiting unless otherwise specified. Thus, the invention should in no way be construed as being limited to the following examples. Rather, the examples should be construed to encompass any and all variations which become evident as a result of the teaching provided herein.

Without further description, it is believed that the skilled person can, using the preceding description, the following illustrative examples and the common general knowledge make use of the present invention. The following working examples point to some of the preferred embodiments of the present invention, and are not to be construed as limiting in any way on the remainder of the disclosure.

### Materials & Methods

### Cell lines and tissue culture

The human melanoma cell lines IRNE and 1359-Mel were kindly provided by G. Pawalec (University Tuebingen, Germany) and have recently been published (Lazaridou, M.F., et al., J Clin Med, 2020. 9(9)).These cell lines were maintained in Roswell Park Memorial Institute 1640 Medium (RPMI, Invitrogen^{™}) supplemented with 10 % (v/v) fetal bovine serum (FCS) (PAA, Pashing, Austria), 0.1 mM non-essential amino acids (Gibco^{®}, Dublin, Ireland), 2 mM L-glutamine (Lonza^{®}, Basel, Switzerland) and 1 % penicillin/streptomycin (v/v; PAA) at 37 °C, 5 % CO2. The HEK293T cell line was acquired from DSMZ and was cultured in DMEM medium (Sigma-Aldrich, Munich, Germany) supplemented with 10 % (v/v) FCS (PAA), 0.1 mM non-essential amino acids (Gibco), 2 mM L-glutamine (Lonza) and 1 % penicillin/streptomycin (v/v; PAA) at 37° C, 5 % CO2.

### Transfection and luciferase (luc) reporter gene assay

For miRNA mimic transfections, melanoma cells were seeded at 1×10⁵ cells/well in 6-well plates and were transfected with miRNA mimics or controls (both from Sigma^{®}, Kawasaki, Japan), respectively, at a final amount of 10 pM using Lipofectamine RNAi MAX (Invitrogen, Carlsbad, CA, USA) according to the manufacturers' reverse transfection protocol.

The following reverse transfection method was used to reverse transfect miRNA mimics into mammalian cells in a 24-well format. In reverse transfections, the complexes are prepared inside the wells, after which cells and medium are added. Reverse transfections are faster to perform than forward transfections, and are the method of choice for high-throughput transfection.

The transfection was optimized for the cell line used. All amounts and volumes are given on a per well basis.
1. For each well to be transfected, RNAi duplex-Lipofectamine RNAiMAX complexes were prepared as follows. 6 pmol RNAi duplex were diluted in 100 µl Opti-MEM I Medium without serum in the well of the tissue culture plate and gently mixed. Lipofectamine RNAiMAX was gently mixed before use, then 1 µl Lipofectamine RNAiMAX was added to each well containing the diluted RNAi molecules, gently mixed and incubated for 10-20 minutes at room temperature.
2. Cells were diluted in complete growth medium without antibiotics so that 500 µl contains the appropriate number of cells to give 30-50% confluence 24 hours after plating. 20,000-50,000 cells/well for suspension cells were used.
3. 500 µl of the diluted cells were added to each well with RNAi duplex - Lipofectamine RNAiMAX complexes. This gave a final volume of 600 µl and a final RNA concentration of 10 nM. The plates were mixed by gently rocking the plates back and forth.
4. The cells were incubated 24-72 hours at 37°C in a CO₂ incubator until ready to assay for gene knockdown.

Cells were harvested 48 hours after transfection and directly subjected to analysis.

For the luciferase (luc) reporter assay, 1×10⁴ HEK293T cells/well were seeded in 96-well plates. After 16 h, 10 ng of the reporter plasmid (pmirGLO Dual-Luciferase miRNA Target Expression Vector, Promega^{™}) in combination with the respective miRNA mimic or control (Sigma) at a final concentration of 25 nM were transfected using Lipofectamine^{™} 2000 (Invitrogen) according to the manufacturers' protocol. Luc activity was determined 48 h after transfection employing the Dual-Glo^{®} Luciferase substrate (Promega) according to the manufacturers' protocol. Luminescence was determined by a Tecan Infinite 200 Pro plate reader device. Relative luc activity was determined by normalizing the firefly reporter to renilla luc activity.

### RNA isolation, cDNA synthesis and quantitative PCR

Total RNA was isolated from cultured cells using the NucleoSpin^{®} miRNA kit (Macherey & Nagel) according to the manufacturers' instructions followed by DNase I treatment (NEB). RNA quality and quantity were assessed by spectrophotometric analysis and 500 ng of total RNA was used for cDNA synthesis (RevertAid H Minus First Strand cDNA synthesis kit, Fermentas, Waltham, MS, USA).

For miRNA-specific cDNA synthesis a miRNA-specific stem-loop primer was used, while reverse transcription of mRNAs required a random hexamer primer (Fermentas). Expression levels were analyzed by qRT-PCR using GoTaq^{®} qPCR Master Mix (Promega). For all primer pairs, an annealing temperature of 60°C was used. Relative changes of mRNA/miRNA amounts were determined by the ΔΔCt method using U6 snRNA, U44 snoRNA, U46 snoRNA and 47sno for normalization. All primers are listed in Table 3 below.

**Table 3. primers**

| **primer name** | **sequence** | **SEQ ID Nos** |
|---|---|---|
| miR-0009-5p qPCR FW | GCCCGCTCTTTGGTTATCTAGC | SEQ ID NO: 65 |
| miR-0009-5p SL RT | GTCGTATCCAGTGCAGGGTCCGAGGTATTCGCACTGGATA CGAC TCATAC | SEQ ID NO: 66 |
| miR-0015a-5p qPCR FW | GCCCTAGCAGCACATAATGG | SEQ ID NO: 67 |
| miR-0015a-5p RT SL | GTCGTATCCAGTGCAGGGTCCGAGGTATTCGCACTGGATA CGAC CACAAA | SEQ ID NO: 68 |
| miR-0016-5p qPCR FW | GCCCCTAGCAGCACGTAAATA | SEQ ID NO: 69 |
| miR-0016-5p SL RT | GTCGTATCCAGTGCAGGGTCCGAGGTATTCGCACTGGATA CGAC CGCCAA | SEQ ID NO: 70 |
| miR-0017-5p qPCR FW | GCCCCAAAGTGCTTACAGTG | SEQ ID NO: 71 |
| miR-0017-5p RT SL | GTCGTATCCAGTGCAGGGTCCGAGGTATTCGCACTGGATA CGAC CTACCT | SEQ ID NO: 72 |
| miR-0025-3p qPCR FW | GCCCCATTGCACTTGTCTCG | SEQ ID NO: 73 |
| miR-0025-3p RT SL | GTCGTATCCAGTGCAGGGTCCGAGGTATTCGCACTGGATA CGAC TCAGAC | SEQ ID NO: 74 |
| miR-0026a RT SL | GTCGTATCCAGTGCAGGGTCCGAGGTATTCGCACTGGATA CGAC AGCCTA | SEQ ID NO: 75 |
| miR-0026a-5p qPCR FW | GCCCTTCAAGTAATCCAGGA | SEQ ID NO: 76 |
| miR-0026b-5p SL RT | GTCGTATCCAGTGCAGGGTCCGAGGTATTCGCACTGGATA CGAC ACCTAT | SEQ ID NO: 77 |
| miR-0027a-3p qPCR FW | CACGCATTCACAGTGGCTAAG | SEQ ID NO: 78 |
| miR-0027a-3p SL RT | GTCGTATCCAGTGCAGGGTCCGAGGTATTCGCACTGGATA CGAC GCGGAA | SEQ ID NO: 79 |
| miR-0027b-3p qPCR FW | GCCCTTCACAGTGGCTAAGT | SEQ ID NO: 80 |
| miR-0027b-3p RT SL | GTCGTATCCAGTGCAGGGTCCGAGGTATTCGCACTGGATA CGAC GCAGAA | SEQ ID NO: 81 |
| miR-0029a-3p qPCR FW | GCCCTAGCACCATCTGAAAT | SEQ ID NO: 82 |
| miR-0029a-3p RT SL | GTCGTATCCAGTGCAGGGTCCGAGGTATTCGCACTGGATA CGAC TAACCG | SEQ ID NO: 83 |
| miR-0029b qPCR FW | GCCCTAGCACCATTTGAAATC | SEQ ID NO: 84 |
| miR-0029b RT SL | GTCGTATCCAGTGCAGGGTCCGAGGTATTCGCACTGGATA CGAC AACACT | SEQ ID NO: 85 |
| miR-0034a qPCR FW | GCCCCAATCAGCAAGTATACTGC | SEQ ID NO: 86 |
| miR-0034a RT SL | GTCGTATCCAGTGCAGGGTCCGAGGTATTCGCACTGGATA CGAC AGGGCA | SEQ ID NO: 87 |
| miR-0034a-5p qPCR FW | GCCCTGGCAGTGTCTTAG | SEQ ID NO: 88 |
| miR-0034a-5p SL RT | GTCGTATCCAGTGCAGGGTCCGAGGTATTCGCACTGGATA CGAC ACAACC | SEQ ID NO: 89 |
| miR-0103a-3p qPCR FW | GCCCAGCAGCATTGTACAGG | SEQ ID NO: 90 |
| miR-0103a-3p RT SL | GTCGTATCCAGTGCAGGGTCCGAGGTATTCGCACTGGATA CGAC TCATAG | SEQ ID NO: 91 |
| miR-0103b-5p qPCR FW | GCCCTCATAGCCCTGTACAA | SEQ ID NO: 92 |
| miR-0103b-5p RT SL | GTCGTATCCAGTGCAGGGTCCGAGGTATTCGCACTGGATA CGAC AGCAGC | SEQ ID NO: 93 |
| miR-0146a-5p qPCR FW | CACGCATGAGAACTGAATTCC | SEQ ID NO: 94 |
| miR-0146a-5p SL RT | GTCGTATCCAGTGCAGGGTCCGAGGTATTCGCACTGGATA CGAC AACCCA | SEQ ID NO: 95 |
| miR-0148a-3p qPCR FW | GCCCTCAGTGCACTACAGAAC | SEQ ID NO: 96 |
| miR-0148a/b-3p RT SL | GTCGTATCCAGTGCAGGGTCCGAGGTATTCGCACTGGATA CGAC ACAAAG | SEQ ID NO: 97 |
| miR-0148b-3p qPCR FW | GCCCTCAGTGCATCACAGAAC | SEQ ID NO: 98 |
| miR-0152-3p qPCR FW | GCCCTCAGTGCATGACAGA | SEQ ID NO: 99 |
| miR-0152-3p RT SL | GTCGTATCCAGTGCAGGGTCCGAGGTATTCGCACTGGATA CGAC CCAAGT | SEQ ID NO: 100 |
| miR-0155-5p qPCR FW | CACGCATTAATGCTAATCGTGAT | SEQ ID NO: 101 |
| miR-0155-5p SL RT | TCGTATCCAGTGCAGGGTCCGAGGTATTCGCACTGGATAC GAC ACCCCT | SEQ ID NO: 102 |
| miR-0181a-5p qPCR FW | CACGCAAACATTCAACGCTGTC | SEQ ID NO: 103 |
| miR-0181a-5p SL RT | TCGTATCCAGTGCAGGGTCCGAGGTATTCGCACTGGATAC GAC ACTCAC | SEQ ID NO: 104 |
| miR-0181b-5p qPCR FW | GCCCAACATTCATTGCTGTC | SEQ ID NO: 105 |
| miR-0181b-5p RT SL | GTCGTATCCAGTGCAGGGTCCGAGGTATTCGCACTGGATA CGAC ACCCAC | SEQ ID NO: 106 |
| miR-0186-5p qPCR FW | GCCCCAAAGAATTCTCCTTT | SEQ ID NO: 107 |
| miR-0186-5p RT SL | GTCGTATCCAGTGCAGGGTCCGAGGTATTCGCACTGGATA CGAC AGCCCA | SEQ ID NO: 108 |
| miR-0200c-3p qPCR FW | GCCCTAATACTGCCGGGTAA | SEQ ID NO: 109 |
| miR-0200c-3p RT SL | GTCGTATCCAGTGCAGGGTCCGAGGTATTCGCACTGGATA CGAC TCCATC | SEQ ID NO: 110 |
| miR-0320a SL RT | GTCGTATCCAGTGCAGGGTCCGAGGTATTCGCACTGGATA CGAC TCGCCC | SEQ ID NO: 111 |
| miR-0320a/b qPCR FW | GCCAAAAGCTGGGTTGAGAG | SEQ ID NO: 112 |
| miR-0324-5p qPCR FW | GAAACGCATCCCCTAGGGCAT | SEQ ID NO: 113 |
| miR-0324-5p RT SL | GTCGTATCCAGTGCAGGGTCCGAGGTATTCGCACTGGATA CGAC ACACCA | SEQ ID NO: 114 |
| miR-0340-5p qPCR FW | GCCCGGTTATAAAGCAATGAGAC | SEQ ID NO: 115 |
| miR-0340-5p RT SL | GTCGTATCCAGTGCAGGGTCCGAGGTATTCGCACTGGATA CGAC AATCAG | SEQ ID NO: 116 |
| miR-0424-5p qPCR FW | CACGCACAGCAGCAATTCATG | SEQ ID NO: 117 |
| miR-0424-5p SL RT | TCGTATCCAGTGCAGGGTCCGAGGTATTCGCACTGGATAC GAC TTCAAA | SEQ ID NO: 118 |
| miR-0516b-5p qPCR FW | GCGCAATCTGGAGGTAAGAAGC | SEQ ID NO: 119 |
| miR-0516b-5p SL RT | GTCGTATCCAGTGCAGGGTCCGAGGTATTCGCACTGGATA CGAC AAAGTG | SEQ ID NO: 120 |
| SL qPCR RV | CCAGTGCAGGGTCCGAGGTA | SEQ ID NO: 121 |
| U6 snRNA qPCR FW | CGGCAGCACATATACTAAAATTGGA | SEQ ID NO: 122 |
| U6 snRNA qPCR RV | AATATGGAACGCTTCACGAATTTGC | SEQ ID NO: 123 |
| RNU44 FW | CCTGGATGATGATAAGCAAATGC | SEQ ID NO: 124 |
| RNU44 RV | CAGTTAGAGCTAATTAAGACCTTC | SEQ ID NO: 125 |
| RNU46 FW | GGGTGATGAAAAAGAATCCTTAGG | SEQ ID NO: 126 |
| RNU46 RV | GTGTAACTATGACAAGTCCTTGC | SEQ ID NO: 127 |
| RNU47 FW | GATGTAATGATTCTGCCAAATG | SEQ ID NO: 128 |
| RNU47 RV | CCTCAGAATCAAAATGGAACG | SEQ ID NO: 129 |

### Flow cytometry

For flow cytometric analyses the following monoclonal antibodies (mAbs) were employed: APC conjugated PD-L1 (clone MIH3, Invitrogen) and FITC conjugated annexin V (Miltenyl Biotech). Cells were stained with the annexin V mAb as recommended by the manufacturers and then stained with the anti-PD-L1 mAb at a 1:100 dilution for 30 min at 4°C. The measurements were performed on a BD LSR Fortessa unit (Becton Dickinson, Heidelberg, Germany). FlowJo software (FlowJo, LLC) was used for analyses and to display flow cytometry results. The results are presented in histograms as mean fluorescence intensity (MFI).

### Western blot analysis

For the quantification of PD-L1 protein expression, total protein from transfected and control cells was extracted and quantified using the Pierce BCA protein assay kit (Thermo Fisher, Waltham, MS, USA). 50 µg of total protein was loaded on 10% denaturing polyacrylamide gels and transferred onto nitrocellulose membranes (GE Healthcare, Chicago, IL, USA). The membranes were blocked in TBS-T, 5%(w/v) skimmed milk for 1h and incubated with the primary antibodies directed against PD-L1 (CAL10 clone, abcam, 1:1000 dilution in TBS-T, 5%(w/v) bovine serum albumin (BSA)) and β-actin (clone, abcam, Cambridge, UK, 1:2000 dilution in TBS-T, 5% (w/v) BSA) overnight at 4°C. For detection, a secondary antibody conjugated with horseradish peroxidase (HRP) and the Lumi-Light substrate (Roche Applied Science) were applied. The signal was recorded with a LAS3000 camera system (Fuji LAS3000, Fuji GmbH, Düsseldorf) using the Image Reader LAS3000 software. The immunostaining signals were subsequently analyzed using the ImageJ software (NIH, Bethesda, MD, USA). Quantification was done by setting the peak values of the control protein (β-actin) for each loaded sample as "1".

### miRNA enrichment assay (miTRAP) and small RNA sequencing

To identify potential miRNAs targeting the 3'-UTR or the CDS of PD-L1, the miTRAP method was employed, as recently described in Friedrich, M. et al., Journal for immunotherapy of cancer 8.1 (2020) and based on Braun, J, et al., Nucleic acids research 42.8 (2014): e66-e66 each of which is incorporated herein in their entireties. The miTRAP eluates were either used for cDNA synthesis of candidate or control miRNAs or subjected to small RNA sequencing (RNA-seq), which was carried out at Novogene (Hong-Kong, China) and analyzed as published (Friedrich, M. et al., Journal for immunotherapy of cancer 8.1 (2020).

### Stimulation of peripheral blood mononuclear cells and cytotoxicity assays

In order to investigate the functional potential of the miRNA-mediated PD-L1 regulation, cytotoxic assays were performed. Briefly, total peripheral blood mononuclear cells (PBMCs) obtained from healthy HLA-A*03 donors were isolated using density gradient centrifugation (Biocoll^{®}, Biochrom GmbH, Berlin, Germany) and cultured in X-VIVO15^{™} medium. Cytotoxic T cells were induced either with a 5-day hyperactivation of the PBMCs with a high concentration of IL-2 (4000 U/mL) or via generation of antigen specific T cells using the following protocol:
PBMC were seeded in 6 well plate at a density of 200.000 cells/well. After 24 h, non-adherent lymphocytes were transferred into new wells and cultured in the presence of 200 U/mL human recombinant IL-2 (Proleukin, Clinigen, Burton upon Trent, UK) whereas adherent monocytes were supplemented for 5 days with 100 ng/mL rh-GM-CSF (Sanofi, Paris, France) and 100 ng/mL rh-IL-4 (Immunotools, Friesoythe, Germany) to induce dendritic cell differentiation. After additional 24 h culture in the presence a lysate from 2x10⁵ 1359-Mel cells obtained with heat shock (3x 2' cycles between liquid nitrogen and 37°C water bath), they were used to stimulate the non-adherent lymphocytes in the presence of small concentration of IL-2 (20 u/mL). After one week, an additional cycle of stimulation with tumor lysate pulsed cells was performed.

For the generation of T cells capable of recognizing the mel1359 cell line, non-adherent cells were removed 24 hours after plating of the PBMCs and cultured separately. Adherent cells were supplemented with 100ng/mL rh-GM-CSF (Sanofi^{®}) and 100ng/mL rh-IL-4 (Immunotools^{®}) for 5 days. On day 5, mel1359 lysate from 2x10⁵ cells treated with heat shock (3x 2' cycles between liquid nitrogen and 37°C water bath) was added. After 24 h, the removed non-adherent cells were added back for the antigen presentation. The cells were used for cytotoxicity assays after 2 consecutive stimulations with pulsed antigen presenting cells.

Due to the low basal expression on CTL obtained with both protocols, PD1 expression was induced 24 h prior to the assay by anti-CD3 (plate coated, 1h at 37°C, eBioscience, San Diego, CA, USA) and anti-CD28 (BD Pharmigen) stimulation in the presence of 20 ng/mL IL-6 (kindly provided by S. Rose-John, Institute of Biochemistry, University of Kiel, Kiel, Germany), 20ng/mL IL-12 (Immunotools^{®}) and 50 ng/mL TGF- β1 (Biolegend^{®}).

Mel1359 melanoma cells were transfected as described above (mock or with miRNA mimics) and stained with CellTrace^{™} CFSE (Invitrogen^{®}) for the mock control and the Cell Proliferation Dye eFluor^{™} 670 (eBioscience^{®}) for the miRNA mimics (miR-17, miR-155, miR-103b and miR-186) according to manufacturer's protocol. The cells (mock and miRNA mimic transfected) were then mixed (at a 1:1 ratio) and further incubated with the previously stimulated effector cells for 4 h at an effector to target (E:T) ratio of 5:1 and 10:1, followed by staining with Zombie aqua (Biolegend^{®}) for viability assessment. A ratio was formed as follows: number of live mock transfected cells in the presence of stimulated PBMCs, divided by the number of live mock transfected cells in the absence of stimulated PBMCS (control see below). The same was done for the miRNA mimic transfected cells: number of live miRNA mimic (miR-17, miR-155, miR-103b or miR-186) transfected cells in the presence of stimulated PBMCs, divided by the number of live miRNA mimic transfected cells in the absence of stimulated PBMCs (control, see below). Incubation of Mel1359 cells with unstimulated PBMCs (cultured with the standard concentration of 200 ng/mL IL-2) from the same donors served as controls.

### Statistical analysis

Mann-Whitney tests, chi-square tests and survival analysis were performed and plotted using GraphPad Prism v.8.0.1. Multivariate cox regression survival analysis was completed in IBM SPSS statistics 25. The data were considered significant with a p value < 0.05.

### Example 1: Identification of PD-L1 targeting miRNA candidates

PD-L1 regulating miRNAs were identified using the miTRAP method (Braun, J., et al., Nucleic Acids Res, 2014. 42(8): p. e66) (Figure 1A), followed by RNA-seq (Tretbar, U.S., et al., Methods Mol Biol, 2019. 1913: p. 81-101. As bait, *in vitro* transcribed RNA representing the CDS and the 3'-UTR sequence, which was split due to its large size into two parts (see Table 2, 3'UTR-1 and 3'UTR-2), were used. Since an important requirement for the outcome of the miTRAP analysis is the selection of a suitable cell line, the PD-L1 mRNA and protein expression profiles of 49 different melanoma cell lines were monitored regarding a discordant PD-L1 expression as indicator of a post-transcriptional regulation.

Based on these data the melanoma cell line IRNE was chosen for miTRAP analysis, since it showed a low surface expression of PD-L1 despite high mRNA levels, which is indicative of a posttranscriptional regulation of PD-L1. Following miRNA enrichment co-purified miRNAs were assessed by high-throughput small RNA-seq. Libraries were generated from the PD-L1 CDS, both parts of the 3'-UTR (3'UTR-1 and 3'UTR-2) and MS2 control miTRAP eluates. The RNA-seq results revealed high levels of co-precipitated miRNAs within the eluates of the different regions investigated (Figure 1B), with 142 unique miRNAs detected. The high reliability of this method was underlined by the fact that more than 75% of the known PD-L1 specific miRNAs were found using this approach (see Table in Figure 1F). 15 of the 142 detected miRNAs were equally found in the eluates of the PD-L1 CDS and both 3' UTRs. The highest number of individually identified miRNAs (n=62) were found in the eluates of the 3' UTR-1 as bait. After evaluation of the normalized read counts (TPM) and setting up stringent criteria for the identification of potential PD-L1-regulating miRNA candidates, such as (i) high normalized read counts and enrichment ratio, (ii) binding sites in at least 2 of the investigated PD-L1 sequences, (iii) multiple binding sites and (iv) binding prediction by at least three out of four applied tools, a number of miRNA candidates potentially regulating PD-L1 were selected. Their expression was validated by qPCR analysis of the miTRAP eluates as presented in Figure 1C-1E.

Of the confirmed miRNAs, 6 miRNAs, namely miR-26a-5p (SEQ ID NO: 1) miR-26b-5p (SEQ ID NO: 2), miR-29a-3p (SEQ ID NO: 3), miR-103b (SEQ ID NO: 4), miR-181b-5p (SEQ ID NO: 5) and miR-186-5p (SEQ ID NO: 6) were chosen to further investigate their contribution in PD-L1 regulation based on expression levels, number of predicted binding sites and immune modulatory functions in the literature. Based on its direct binding on PD-L1 mRNA as well as the regulation of c-Fos, a known transcription factor of PD-L1, miR-155-5p was included in the analysis despite absence of detection in the miTRAP eluates (low/no expression in the chosen IRNE cell line). In addition, miR-17-5p was also included as a positive control in the assays.

A selected summary of miRNA characteristics is provided in Table 4 below. A selected summary of the miRNA read counts obtained for the PD-L1 CDS and MS2 control are provided in Table 5.

| **Table 4.** Summary of miRNA characteristics | | | | | | |
|---|---|---|---|---|---|---|
| | **CDS** | | **3'-UTR-1** | | **3'-UTR-2** | |
| | Number of unique binding sites | seed region position | Number of unique binding sites | seed region position | Number of unique binding sites | seed region position |
| **mir-17-5p** | 0 | | 0 | | 1 | 3437-3443 |
| **miR-29a-3p** | 0 | | 1 | 1557-1561 | 1 | 2986-2992 |
| **miR-103b** | 1 | 419-425 | 1 | 1977-1982 | 0 | |
| **miR-155-5p** | 0 | | 0 | | 2 | 2276-2282, 3528-3534 |
| **miR-181b-5p** | 0 | | 1 | 1625-1630 | 1 | 3576-3581 |
| **miR-186-5p** | 1 | 85-90 | 2 | 1454-1459, 1930-1935 | 3 | 2698-2683, 3011-3016, 3333-3339 |

**Table 5. Read counts for CDS, 3' UTR1 and 3'UTR-2 for selected miRNAs.**

| **miRNA** | **CDS** | **MS2 control** | **fold enrichment** |
|---|---|---|---|
| hsa-miR-584-5p | 9834.6 | 114.9 | 85.6 |
| hsa-miR-9-5p | 15423.9 | 206.9 | 74.6 |
| hsa-miR-550a-3-5p | 1429.3 | 23.0 | 62.2 |
| hsa-miR-550a-5p | 1429.3 | 23.0 | 62.2 |
| hsa-miR-454-3p | 1898.7 | 46.0 | 41.3 |
| hsa-miR-27b-3p | 69311.7 | 1792.8 | 38.7 |
| hsa-let-7g-5p | 14677.3 | 436.7 | 33.6 |
| hsa-miR-181a-5p | 6720.0 | 206.9 | 32.5 |
| hsa-miR-122-5p | 1685.3 | 69.0 | 24.4 |
| hsa-miR-3591-3p | 1685.3 | 69.0 | 24.4 |
| hsa-miR-20a-5p | 7829.3 | 321.8 | 24.3 |
| hsa-miR-27a-3p | 51626.4 | 2413.4 | 21.4 |
| hsa-miR-103a-3p | 23039.9 | 1172.2 | 19.7 |
| hsa-miR-185-3p | 1173.3 | 69.0 | 17.0 |
| hsa-miR-25-5p | 1514.7 | 91.9 | 16.5 |
| hsa-miR-222-3p | 18858.6 | 1402.1 | 13.5 |
| hsa-miR-629-5p | 4608.0 | 344.8 | 13.4 |
| hsa-miR-4521 | 8960.0 | 735.5 | 12.2 |
| hsa-let-7e-5p | 5781.3 | 482.7 | 12.0 |
| hsa-miR-211-5p | 2773.3 | 252.8 | 11.0 |
| hsa-miR-221-3p | 7829.3 | 758.5 | 10.3 |
| hsa-let-7f-5p | 10773.3 | 1264.2 | 8.5 |
| hsa-miR-128-3p | 3626.7 | 505.7 | 7.2 |
| hsa-miR-93-5p | 2538.7 | 367.8 | 6.9 |
| hsa-let-7i-5p | 10602.6 | 1563.0 | 6.8 |
| hsa-let-7c-5p | 3840.0 | 597.6 | 6.4 |
| hsa-miR-23a-3p | 1834.7 | 390.7 | 4.7 |
| hsa-miR-378a-3p | 4373.3 | 1011.3 | 4.3 |
| hsa-miR-105-5p | 1152.0 | 298.8 | 3.9 |
| hsa-let-7b-5p | 7040.0 | 2183.6 | 3.2 |
| hsa-miR-193a-5p | 4458.6 | 1448.1 | 3.1 |
| hsa-miR-451a | 1770.7 | 620.6 | 2.9 |
| hsa-miR-103a-2-5p | 1130.7 | 0.0 | 0 |
| hsa-miR-550b-3p | 1237.3 | 0.0 | 0 |

A selected summary of the miRNA read counts obtained for the PD-L1 3'UTR-1 and MS2 control are provided in Table 6.

**Table 6. Read counts for the 3' UTR1 for selected miRNAs.**

| **miRNA** | **3'UTR-1** | **MS2 control** | **fold enrichment** |
|---|---|---|---|
| hsa-miR-140-5p | 26822.2 | 207.7 | 129.1 |
| hsa-miR-425-5p | 6937.1 | 115.4 | 60.1 |
| hsa-miR-30b-5p | 1312.8 | 23.1 | 56.9 |
| hsa-miR-574-3p | 2125.8 | 92.3 | 23.0 |
| hsa-miR-29b-3p | 1759.3 | 92.3 | 19.1 |
| hsa-miR-9-5p | 3485.2 | 207.7 | 16.8 |
| hsa-miR-320b | 9669.3 | 692.4 | 14.0 |
| hsa-miR-25-3p | 6217.4 | 623.1 | 10.0 |
| hsa-miR-103a-3p | 11675.2 | 1177.0 | 9.9 |
| hsa-miR-320c | 1572.7 | 184.6 | 8.5 |
| hsa-miR-629-5p | 2419.0 | 346.2 | 7.0 |
| hsa-miR-24-3p | 17179.6 | 3208.0 | 5.4 |
| hsa-miR-3074-5p | 16853.0 | 3184.9 | 5.3 |
| hsa-miR-532-3p | 1186.2 | 230.8 | 5.1 |
| hsa-miR-181a-5p | 1039.6 | 207.7 | 5.0 |
| hsa-miR-27b-3p | 7223.7 | 1800.2 | 4.0 |
| hsa-miR-30c-5p | 5890.9 | 1546.3 | 3.8 |
| hsa-miR-151a-3p | 5977.5 | 1707.9 | 3.5 |
| hsa-miR-4521 | 2199.1 | 738.5 | 3.0 |
| hsa-miR-106b-3p | 1572.7 | 530.8 | 3.0 |
| hsa-miR-23a-3p | 1126.2 | 392.3 | 2.9 |

**Table 7. Read counts for the 3' UTR2 for selected miRNAs.**

| **miRNA** | **3'UTR-2** | **MS2** | **fold enrichment** |
|---|---|---|---|
| hsa-miR-20a-5p | 40453.9 | 94.7 | 427.4 |
| hsa-miR-146b-5p | 7683.5 | 74.4 | 103.3 |
| hsa-miR-3074-5p | 11949.7 | 933.1 | 12.8 |
| hsa-miR-24-3p | 12011.0 | 939.8 | 12.8 |
| hsa-miR-151a-3p | 4772.8 | 500.4 | 9.5 |
| hsa-miR-30e-5p | 1281.1 | 209.6 | 6.1 |
| hsa-miR-103a-3p | 1190.8 | 344.8 | 3.5 |
| hsa-miR-222-3p | 1394.1 | 412.5 | 3.4 |
| hsa-miR-340-5p | 364.7 | 108.2 | 3.4 |

**Table 8. List of known miRNA found in miTRAP eluates.**

| **known PD-L1 miRNAs (according to PMID: 31258527, 29186904)** | **found in our miTRAP eluates?** |
|---|---|
| miR-15a | yes |
| miR-15b | yes |
| miR-16 | yes |
| miR-17-5p | yes |
| miR-18a | yes |
| miR-33a | yes |
| miR-34a | yes |
| miR-93-5p | yes |
| miR-106-5p | yes |
| miR-138-5p | no |
| miR-140 | yes |
| miR-142-5p | no |
| miR-152 | yes |
| miR-155 | no |
| miR-193a-3p | no |
| miR-195 | yes |
| miR-200 | yes |
| miR-324-5p | yes |
| miR-338-5p | yes |
| miR-340 | yes |
| miR-383 | no |
| miR-424 | yes |
| miR-513 | no |
| **total** | **0.773** |

**Table 9. List of miRNA of the invention and target site location.**

| **miRNA** | **CDS** | **3' UTR-1** | **3' UTR-2** |
|---|---|---|---|
| hsa-miR-103a-3p | + | + | + |
| hsa-miR-103b | + | + | |
| hsa-miR-107 | + | + | + |
| miR186-5P | + | + | + |
| miR29a-3P | | + | + |
| miR-181b-5P | | + | + |
| hsa-miR-148a-3p | + | + | + |
| hsa-miR-148b-3p | + | + | + |
| hsa-miR-152-3p | + | + | + |
| hsa-miR-181a-5p | + | + | + |
| hsa-miR-181b-5p | + | + | + |
| hsa-miR-211-5p | + | + | + |
| hsa-miR-23a-3p | + | + | + |
| hsa-miR-25-5p | + | + | + |
| hsa-miR-34a-5p | + | + | + |
| hsa-miR-454-3p | + | + | + |
| hsa-miR-584-5p | + | + | + |
| hsa-miR-629-5p | + | + | + |
| hsa-miR-105-5p | + | | + |
| hsa-miR-17-5p | + | | + |
| hsa-miR-20a-5p | + | | + |
| hsa-miR-222-3p | + | | + |
| hsa-miR-451a | + | | + |
| hsa-miR-486-3p | + | | + |
| hsa-miR-486-5p | + | | + |
| hsa-miR-93-5p | + | | + |
| hsa-miR-185-3p | + | + | |
| hsa-miR-23b-3p | + | + | |
| hsa-miR-27a-3p | + | + | |
| hsa-miR-27a-5p | + | + | |
| hsa-miR-27b-3p | + | + | |
| hsa-miR-4521 | + | + | |
| hsa-miR-9-5p | + | + | |
| hsa-miR-135b-5p | | + | + |
| hsa-miR-140-5p | | + | + |
| hsa-miR-151a-3p | | + | + |
| hsa-miR-24-3p | | + | + |
| hsa-miR-26b-5p | | + | + |
| hsa-miR-27b-5p | | + | + |
| hsa-miR-3074-5p | | + | + |
| hsa-miR-340-5p | | + | + |
| hsa-miR-509-3p | | + | + |

### Example 2: Regulatory effect of selected miRNAs on PD-L1 protein level

In order to determine the possible regulatory effects of the identified miRNAs on PD-L1 expression *in vitro,* melanoma cells were transfected with selected miRNA mimics. The cell line 1359-Mel was chosen, due to its high transfection efficiency and moderate to high levels of both mRNA and protein PD-L1 expression to minimize possible preexisting post-transcriptional regulation. Downregulation of PD-L1 surface expression was found upon transfection with miRNA mimics, which ranged from about 10 to about 30% downregulation (Figure 2A). Using flow cytometry, transfection of miR-103b and miR-181b caused reduced PD-L1 expression between about 10% and about 20%, miR-186 overexpression downregulated PD-L1 expression up to about 25%. These results were confirmed by Western blot of transfectants demonstrating a reduced surface expression of PD-L1 (Figure 2B).

### Example 3: Assessment of miRNA binding on selected PD-L1 sequences

In order to confirm whether the observed downregulation of PD-L1 expression upon transfection with the candidate miRNAs is a direct effect of their binding to the PD-L1 3'-UTR or CDS dual luciferase (luc) assays were conducted. The assays were carried out for the miRNAs with *in silico* predicted binding sites and their respective PD-L1 regions (Figure 3A). miR-155-5P reduced the activity > 60% compared to the mock control. miR-181b lead to an equal signal reduction of approximately 30% in both 3'-UTR segments (Figure 3B). Furthermore, miR-103b transfectants demonstrated the second highest decrease of in luc activity of almost 50% targeting the CDS (Figure 3B). The biding site for multiple regions of selected miRNA of the invention is shown in Figure 3C.

In conclusion, it has been shown that the downregulation of PD-L1 by the miRNAs of the invention is a direct effect of the binding of the miRNAs to the CDS, 3'UTR-1 and/or 3'UTR-2 regions of the PD-L1 mRNA.

### Example 4: Function of PD-L1-mediated downregulation

It was investigated whether the miRNA-induced PD-L1 downregulation could enhance T cell-mediated cytotoxicity. Due to the low basal PD1 surface expression on T cells (Figure 4A), T cells were incubated with a cocktail of stimulants (anti-CD3/anti-CD28, IL-6, IL-12, TGF- β1) resulting in a 50% increase in the expression of PD-1 (Figure 4B). As expected, PBMCs hyperstimulated with IL-2 showed lower cytotoxicity compared to the PBMCs primed with the tumor lysate (Figure 4B). In most cases the E:T ratio of 5:1 showed the most prominent differences between mock transfected (FITC) and cells transfected with miR-17 or miR-155 mimics (Figure 4C). Using the primed PBMCs, the ratio of miR-17_{APC}/mock_{FITC} cells showed an approximately 30% and 50% reduction compared to the 3% and 30% reduction of the mock_{FITC} cells, at the E:T ratio of 5:1 and 10:1, respectively (Figure 4C). The strongest cytotoxic effect for miR-155 transfected cells was at an E:T ratio of 10:1 with a 15% increased killing of the miR-155APC cells compared to the mockFITC (72% miR-155APC/alive to 86% mock_{FITC}) (Figure 4C). Similar results were observed for miR-103b and miR-186 (see Figure 4D).

### Example 5: Expression of PD-L1 regulating miRNA candidates on melanoma samples

Formalin-Fixed Paraffin-Embedded (FFPE) samples from 46 melanoma patients were collected at the University Hospital of Zurich, Switzerland. The clinicopathological characteristics of the patients are summarized in Figure 5B. Evaluation of miRNA expression highlighted that miR-29a (SEQ ID NO: 3), miR-181b (SEQ ID NO: 5) and miR-17-5P (SEQ ID NO: 8) had consistently higher expression levels in all melanoma samples in comparison to miR-155-5P (SEQ ID NO: 7) and miR-186-5P (SEQ ID NO: 6), the miRNAs (Figure 5A). By comparing the miRNA expression pattern with PD-L1 positivity, a slightly lower expression of miR-29a (SEQ ID NO: 3) and miR-181b (SEQ ID NO: 5) was detected on PD-L1⁺ melanoma samples (Figure 5A). In order to determine a possible differential regulation of the miRNAs between PD-L1⁻ and PD-L1⁺ patients, a correlation matrix was generated.

Despite some correlations were found in all patients regardless of their PD-L1 status, PD-L1⁺ melanoma lesions showed a stronger correlation between miR-186 and miR-155/miR-181b, while miR-29a and miR-17 were more strongly correlated in PD-L1⁻ patients. Some correlations unique to PD-L1⁺ lesions, such as e.g. miR-181b-5P, appeared to have strong association with all investigated miRNAs. These results therefore show that multiple miRNAs of the invention are related by common nodes of regulation. The simultaneous expression or overexpression of miRNAs belonging to same or different nodes of regulation is therefore expected to provide advantageous effects.

In conclusion, the differential correlations observed are an indication of an altered microenvironment on the PD-L1+ lesions, represented by immune subpopulations expressing the miRNAs of interest, or more importantly suggest the contribution of these miRNAs in a common pathway, which indirectly supports PD-L1 mediated immune escape through our targets of interest.

### Discussion

The Examples show that miRNAs targeting both the CDS and the 3'-UTR of the PD-L1 mRNA have been identified by using the miTRAP technology in combination with RNA sequencing of the eluate. This strategy allowed not only the identification of already known PD-L1-specific miRNAs *in silico* and predicted candidate miRNAs, but also a large number of novel miRNAs has been surprisingly identified. From the various miRNAs with different degrees of enrichment in the miTRAP protocol, it was shown that the miRNAs modulate PD-L1 protein expression in the reporter assay. miRNAs that consistently downregulated the PD-L1 protein expression were identified, which could be used for therapy. Finally, miRNAs targeting directly PD-L1 mRNA and simultaneously regulating other factors of the PD-L1 pathway (e.g. miR-155 and miR-181b with c-Fos) appear to have the strongest effect on PD-L1 expression highlighting the importance of these miRNAs and possible therapeutic approaches. This is further underlined by the effect of miR-155 and other tumor suppressive miRNAs in tumor cells.

## Claims

1. A nucleic acid comprising a sequence that has at least 70%, at least 75%, at least 80%, at least 85%, at least 90%, at least 95% or 100% sequence identity with a sequence selected from the group consisting of the sequences according to any one of SEQ ID NOs: 1 to 60, preferably the sequence is selected from the group consisting of hsa-mir-26a-5p (SEQ ID NO: 1), hsa-miR-26b-5p (SEQ ID NO: 2), hsa-miR-29a-3p (SEQ ID NO: 3), hsa-miR-103b (SEQ ID NO: 4), hsa-miR-181b-5p (SEQ ID NO: 5) and hsa-miR-186-5p (SEQ ID NO: 6), more preferably the sequence is selected from the group consisting of hsa-miR-103b (SEQ ID NO: 4), hsa-miR-181b-5p (SEQ ID NO: 5) and miR-186-5p (SEQ ID NO: 6).

2. The nucleic acid of claim 1, wherein the nucleic acid or a miRNA that is encoded by the nucleic acid is capable of hybridizing to a PD-L1 mRNA.

3. The nucleic acid of claims 1 or 2, wherein the nucleic acid or a miRNA that is encoded by the nucleic acid is capable of hybridizing to the 3' UTR and/or the CDS of a PD-L1 mRNA, preferably wherein the nucleic acid or a miRNA that is encoded by the nucleic acid is capable of hybridizing to both the 3' UTR and the CDS of a PD-L1 mRNA.

4. The nucleic acid of any one of claims 1 to 3, wherein the nucleic acid or a miRNA that is encoded by the nucleic acid by the nucleic acid is capable of reducing gene expression of PD-L1, preferably the nucleic acid or a miRNA that is encoded by the nucleic acid reduces translation of the PD-L1 mRNA.

5. The nucleic acid of any one of claims 1 to 4, wherein the nucleic acid is comprised in a vector.

6. The nucleic acid of any one of claims 1 to 5, wherein the nucleic acid is a miRNA, preferably wherein the nucleic acid is a miRNA mimic.

7. A vector encoding the nucleic acid of any one of claims 1 to 6.

8. A miRNA mimic comprising a sequence that has at least 70%, at least 75%, at least 80%, at least 85%, at least 90%, at least 95% or 100% sequence identity with a sequence selected from the group consisting of the sequences according to any one of SEQ ID NOs: 1 to 60, preferably
a) the miRNA mimic is capable of hybridizing to a PD-L1 mRNA, and/or
b) the miRNA mimic is capable of reducing gene expression of PD-L1, more preferably the miRNA mimic reduces translation of the PD-L1 mRNA.

9. The miRNA mimic of claim 8, wherein the miRNA mimic is capable of hybridizing to the 3' UTR and/or the CDS of a PD-L1 mRNA, preferably wherein the miRNA mimic is capable of hybridizing to both the 3' UTR and the CDS of a PD-L1 mRNA.

10. A cell comprising the nucleic acid of any one of claims 1 to 6, the vector of claim 7 or the miRNA mimic of claims 8 or 9, preferably the cell is an immune cell, preferably a CAR T cell.

11. The nucleic acid of any one of claims 1 to 6, the vector of claim 7, the miRNA mimic of claims 8 or 9 or the cell of claim 10 for use in therapy.

12. The nucleic acid of any one of claims 1 to 6, the vector of claim 7, the miRNA mimic of claims 8 or 9 or the cell of claim 10 for use in treating cancer, preferably the cancer is a skin cancer, non-small cell lung cancer (NSCLC), Hodgkin's lymphoma, bladder cancer, renal cell carcinoma (RCC), head and neck squamous cell carcinoma (HNSCC), breast cancer, Merkel cell carcinoma, hepatocellular carcinoma (HCC) and gastric cancer (GC), more preferably the cancer is skin cancer, melanoma, non-small cell lung cancer (NSCLC), renal cell carcinoma (RCC), bladder cancer and head and neck squamous cell carcinoma (HNSCC), more preferably the cancer is skin cancer, even more preferably the skin cancer is melanoma.

13. Use of the nucleic acid of any one of claims 1 to 6, the vector of claim 7, the miRNA mimic of claims 8 or 9 or the cell of claim 10 for silencing PD-L1 gene expression.

14. The nucleic acid, the vector, the miRNA mimic or the cell for use according to claim 12, wherein the sequence is hsa-miR-155-5p (SEQ ID NO: 7) or hsa-miR-17-5p (SEQ ID NO: 8) or both.

15. A pharmaceutical composition comprising at least one of the nucleic acid of any one of claims 1 to 6, the vector of claim 7, the miRNA mimic of claims 8 or 9 or the cell of claim 10 in combination with a pharmaceutically acceptable carrier or excipient.
